# FASCICULE DE BREVET EUROPEEN

(11) **EP 0 709 383 B1**
(45) Date de publication et mention de la délivrance du brevet: **15.12.1999**
(21) Numéro de dépôt: 95402374.3
(22) Date de dépôt: 25.10.1995
(51) Int. Cl.: C07D 311/30, C07D 407/12, C07H 15/26, C07D 407/14, A61K 31/35

(54) **Dérivés de la diosmétine, leur procédé de préparation et les compositions pharmaceutiques les contenant**
Diosmetin Derivate, Verfahren zu deren Herstellung und diese enthaltende pharmazeutische Zusammenstellungen
Diosmetin derivatives, process for their preparation and pharmaceutical compositions containing them

(30) Priorité: 26.10.1994 FR 9412783
(43) Date de publication de la demande: 01.05.1996
(73) Titulaire: ADIR ET COMPAGNIE, 92415 Courbevoie Cédex (FR)
(72) Inventeur: Wierzbicki, Michel, F-78620 L'Etang la Ville (FR); Boussard, Marie-Françoise, Montainville 78124 Mareil sur Mauldre (FR); Verbeuren, Tony, F-78540 Vernouillet (FR); Vallez, Marie-Odile, F-77420 Champs sur Marne (FR); Canet, Emmanuel, F-75005 Paris (FR); Rolland, Yves, F-92170 Vanves (FR)
(74) Mandataire: Reverbori, Marcelle

(56) Documents cités:
- CH-A- 644 374
- FR-A- 2 701 261
- CHEMICAL ABSTRACTS, vol. 116, no. 23, 1992, Columbus, Ohio, US; abstract no. 231895a, KITANAKA SUSUMU ET AL. 'STUDIES ON THE CONSTITUENTS OF PURGATIVE CRUDE DRUGS.XXIX.' page 456 ; & CHEM. PHARM. BULL., vol.40, no.1, 1992, JAPAN pages 249 - 251
- CHEMICAL ABSTRACTS, vol. 51, no. 10, 1957, Columbus, Ohio, US; abstract no. 8082a, T.H. SIMPSON 'PROTECTING GRUOPS IN THE SYNTHESIS OF FLAVONES.' & SCI. PROC. ROY. DUBLIN SOC., vol.27, 1956, DUBLIN pages 111 - 117

## Description

La présente invention a pour objet les nouveaux dérivés de la diosmétine, leur procédé de préparation et les compositions pharmaceutiques les contenant.

Elle concerne plus particulièrement les composés de formule I : 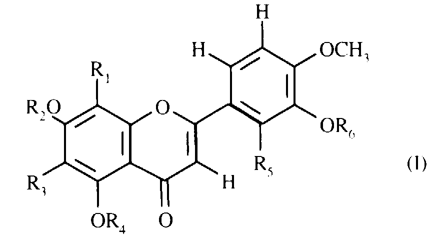 dans laquelle :
- R₁ représente un atome d'hydrogène ou un radical propyle allyle ou 1,2-dideutéroallyle ;
- R₂ représente un atome d'hydrogène ou un radical propyle, allyle, propargyle, 2,3-dihydroxypropyle, (2,2-diméthyl-1,3-dioxol-4-yl) méthyle ou 3-acétyloxy-2-hydroxypropyle ;
- R₃ représente un atome d'hydrogène ou un radical propyle, allyle, ou 1,2-dideutéroallyle ;
- R₄ représente un atome d'hydrogène ou un radical méthyle, propyle, allyle, propargyle, 2,3-dihydroxypropyle, (2,2-diméthyl-1,3-dioxol-4-yl) méthyle ou un radical de formule -COR'₄ [dans laquelle R'₄ représente un radical alkyle de 1 à 5 atomes de carbone en chaîne droite ou ramifiée ou un radical phényle];
- R₅ représente un atome d'hydrogène ou un radical propyle, allyle, ou 1,2-dideutéroallyle, et
- R₆ représente un atome d'hydrogène ou un radical méthyle, propyle, allyle, propargyle, 2.3-dihydroxypropyle, (2,2-diméthyl-1,3-dioxol-4-yl) méthyle, un radical de formule -COR'₆ [dans laquelle R'₆ représente un radical alkyle de 1 à 5 atomes de carbone en chaîne droite ou ramifiée ou un radical phénylel ou un radical de formule A : 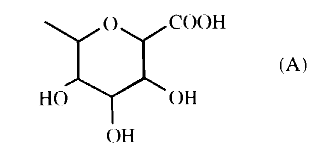 à condition que :
   - l'un au moins des R₁, R₂, R₃, R₄, R₅ et R₆ prenne une signification autre qu'hydrogène, et que
   - si R₁, R₂ et R₃ représentent chacun simultanément un atome d'hydrogène, alors R₄ représente aussi un atome d'hydrogène ;
   - si R₁, R₂, R₃, R₄ et R₅ représentent chacun simultanément un atome d'hydrogène, alors R₆ ne représente pas le radical de formule A ;
ainsi que leurs diastéréoisomères et/ou énantiomères, quand ils existent.

Certains composés de formule I existent sous plusieurs formes cristallines, dont certaines sont particulièrement intéressantes dans le cas où le principe actif est administré sous forme solide (comprimé, suspension ou suppositoire). Ces différentes formes font, à ce titre, partie de la présente invention.

L'état antérieur de la technique est illustré notamment par les brevets : EP 0 319 412 qui concerne des dérivés flavonoïdes 2-pipérazinyl 2-oxo éthylène substitués, utilisables pour le traitement des affections vasculaires, FR 2701261 qui concerne des dérivés glucuronidés de la diosmétine, CH 644374 qui concerne des dérivés hydroxy-éthers de la diosmétine, ainsi que par les publications Chem. Pharm. Bull. 1992, 40(1), 249-251 et Sci. Proc. Roy. Dublin Soc. 1956, Vol. 27, 111-117, qui concernent des dérivés glycosylés de la diosmétine.
Or la prolifération, sans cesse accrue, de ces affections justifie un accueil favorable à toute invention de médicaments dans ce domaine. C'est dans ce cadre que s'insère la présente invention qui concerne une famille de produits de structures chiimiques bien différenciées par rapport à celles des principes actifs antérieurement connus et qui présentent des propriétés pharmacologiques et thérapeutiques particulièrement intéressantes pour le traitement de ces dites affections.

La présence d'une microangiopathie capillaire-veinulaire spécifique a été mise en évidence dans les insuffisances veineuses chroniques. Cette microangiopathie, qui est la conséquence de l'hypertension veineuse, entraîne des troubles de filtration capillaire-veinulaire (hyperperméabilité) et donc des micro-oedèmes (Barbier et al., La Presse Médicale. 23: p 213-224, 1994). L'amélioration des troubles micro circulatoires qui accompagnent l'insuffisance veineuse chronique (oedème, inflammation) doit faire partie du traitement médicamenteux de cette maladie (Chauveau, La Presse Médicale, 23: p243-249, 1994).
Or, il a été trouvé, dans les services de la demanderesse, que les composés objet de la présente invention possèdent non seulement une activité anti-inflammatoire -mise en évidence sur des modèles classiques d'inflammation- mais aussi et surtout une activité anti-hyperperméabilité mise en évidence en utilisant des techniques modernes en microscopie afin d'évaluer les réponses de la micro circulation, cf Bjork et al., Progr. Appl. Microcircul., 6, 41-53. (1984).
Ainsi. les composés de la présente invention sont utilisables notamment dans le traitement de l'insuffisance veineuse chronique.

La présente invention a également pour objet les compositions pharmaceutiques contenant comme principe actif un composé de formule I ou un de ses sels physiologiquement tolérable, mélangé ou associé à un ou plusieurs excipients pharmaceutiques appropriés.

Les compositions pharmaceutiques ainsi obtenues se présentent généralement sous forme dosée renfermant de 1 à 500 mg de principe actif. Elles peuvent. par exemple, revêtir la forme de comprimés, dragées, gélules, suppositoires, solutions injectables ou buvables et être administrées par la voie orale, rectale ou parentérale.
La posologie peut varier notablement selon l'âge et le poids du patient, la voie d'administration, la nature de la maladie et les traitements associés et s'échelonne de 1 à 500 mg de principe actif, 1 à 6 fois par jour.

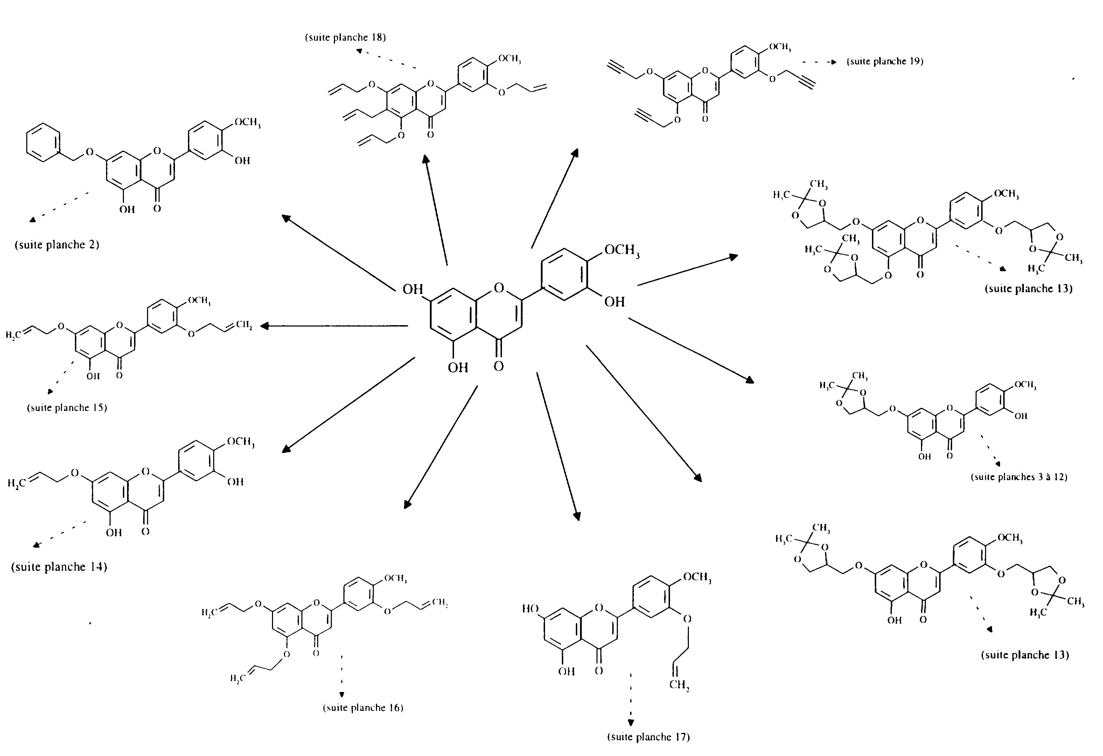

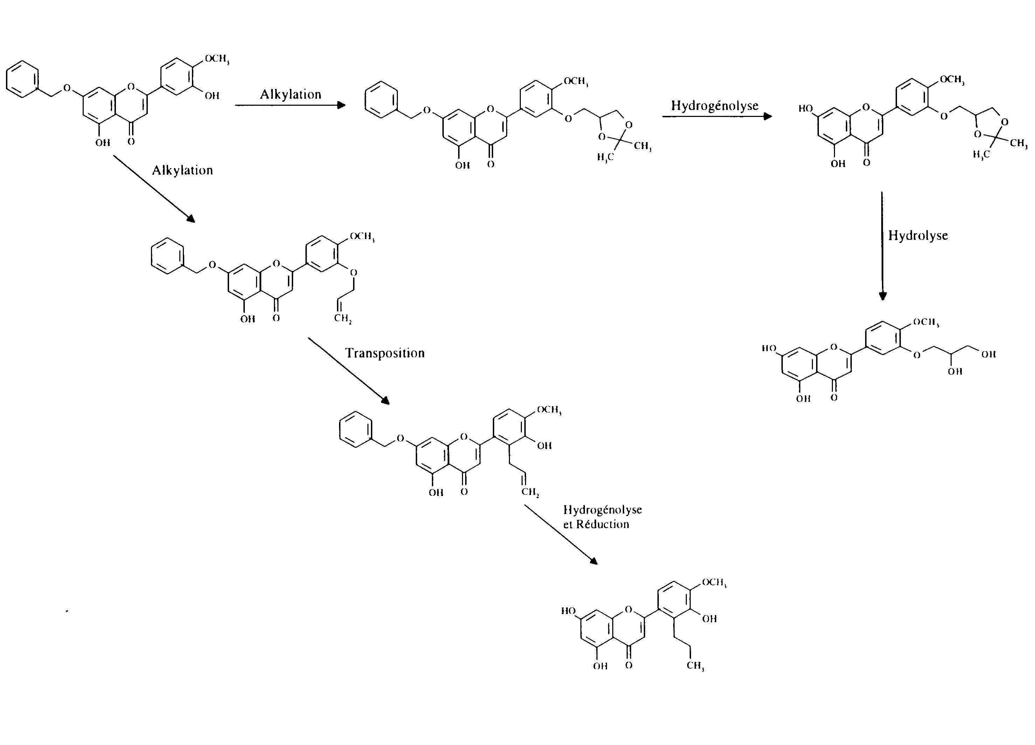

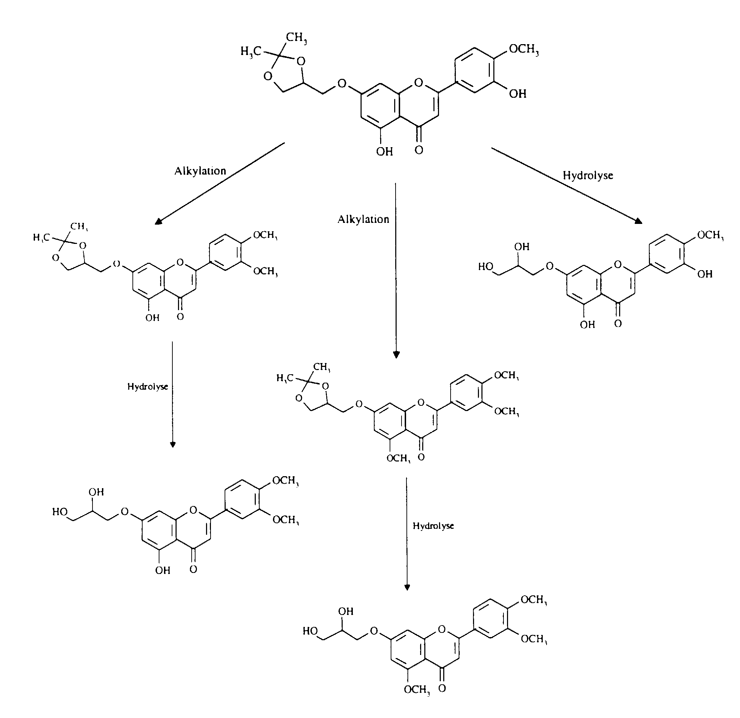

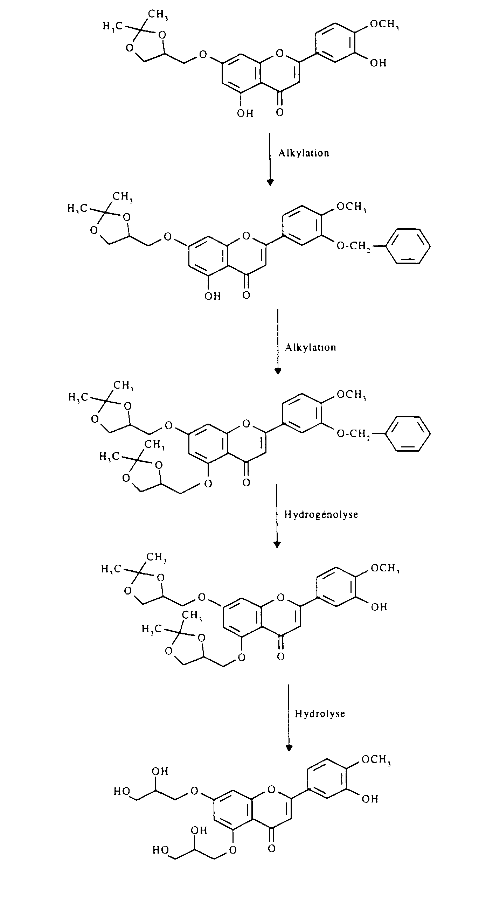

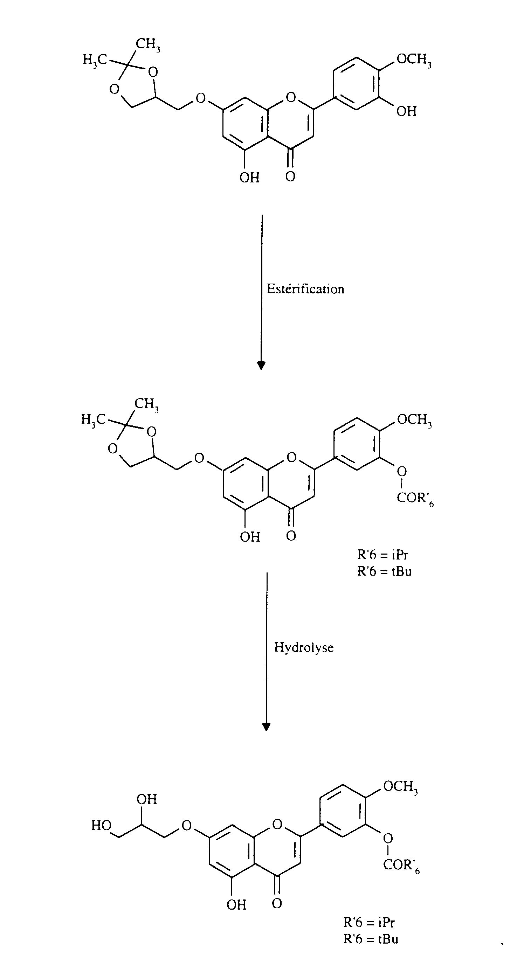

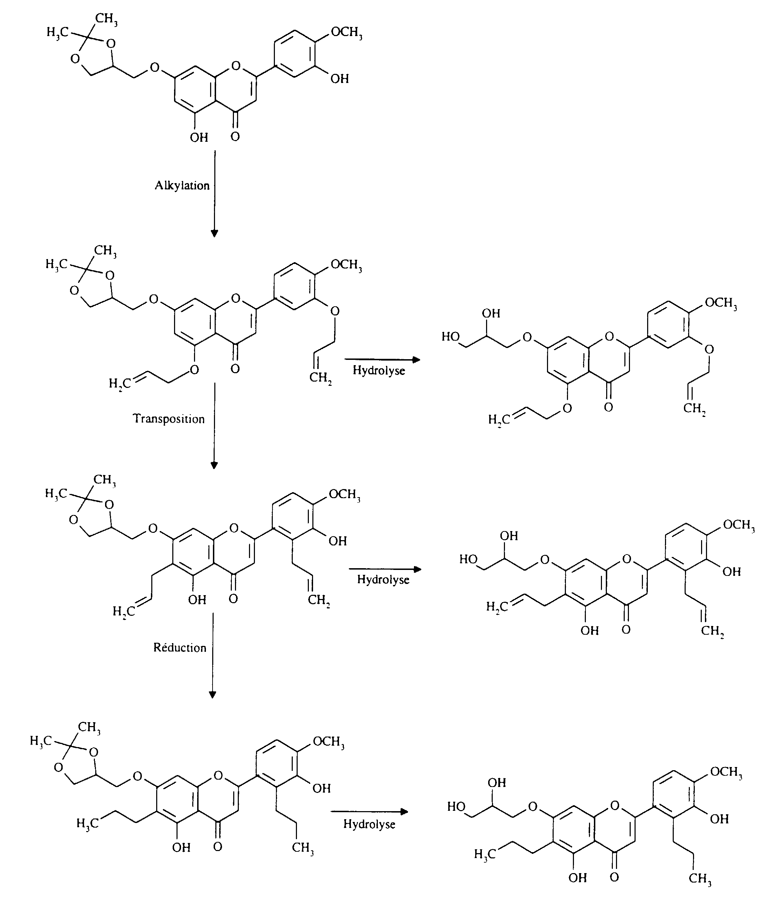

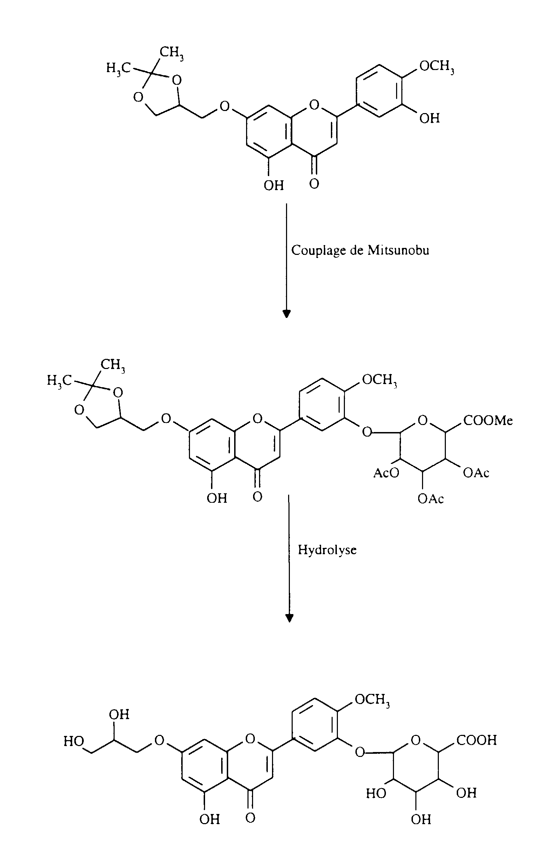

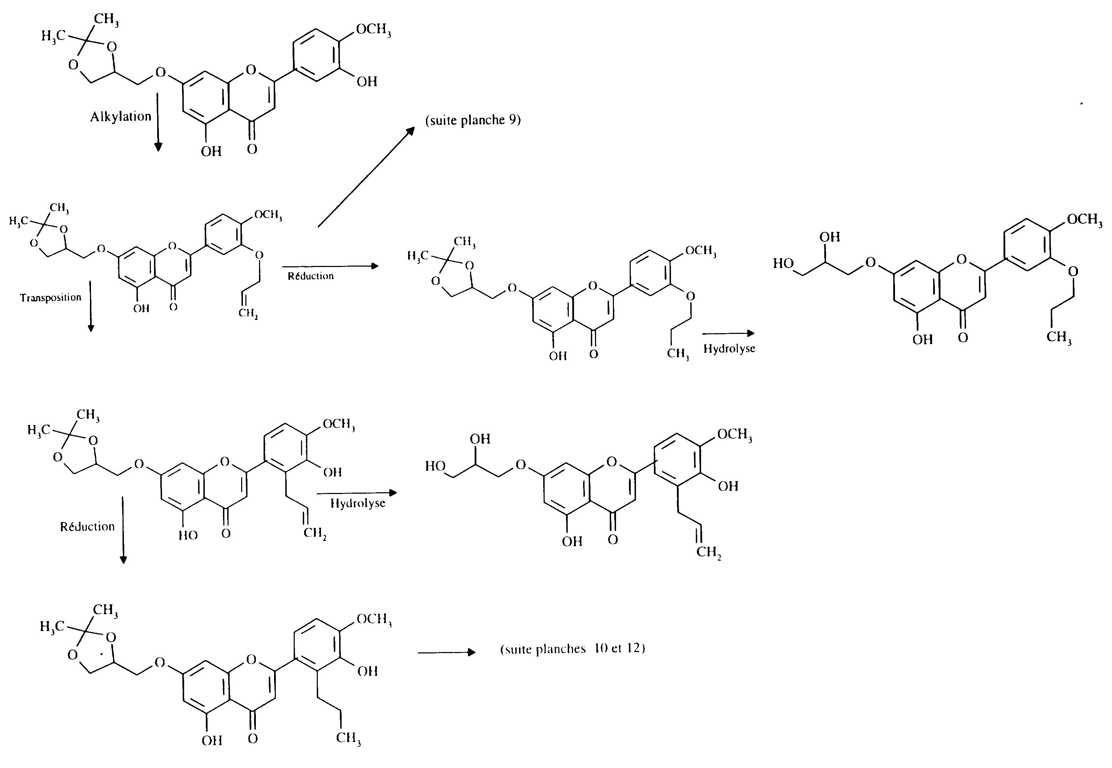

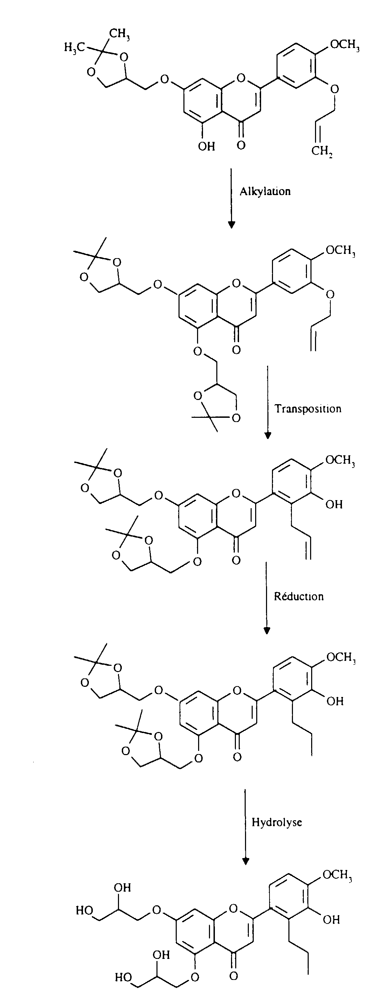

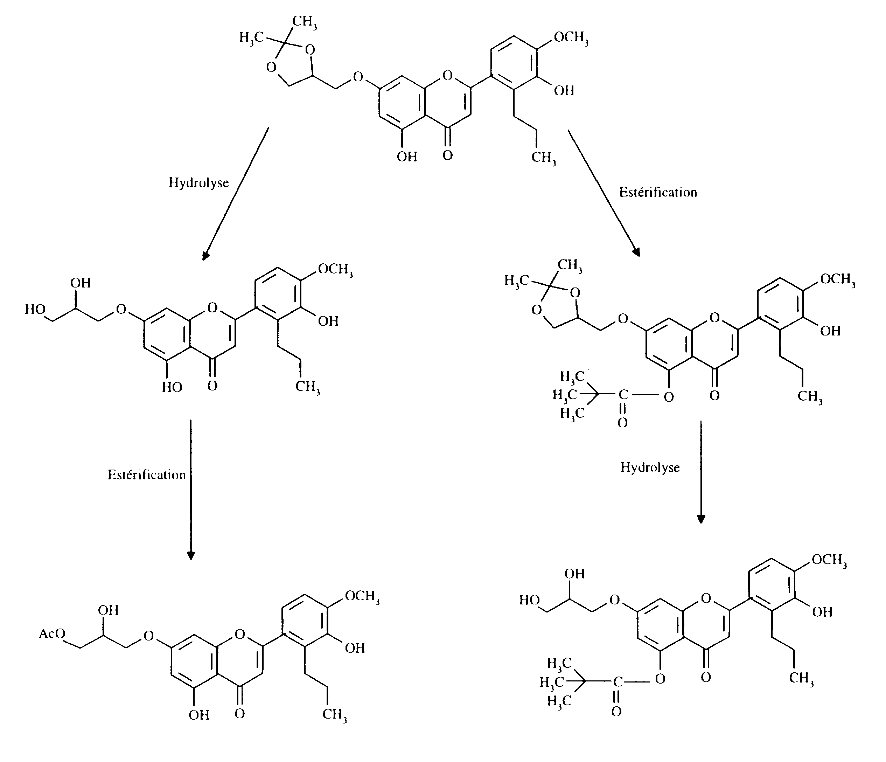

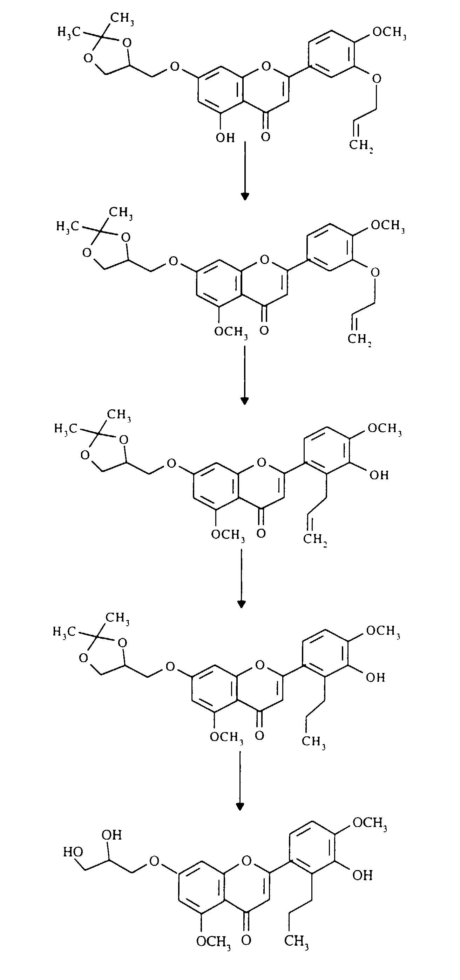

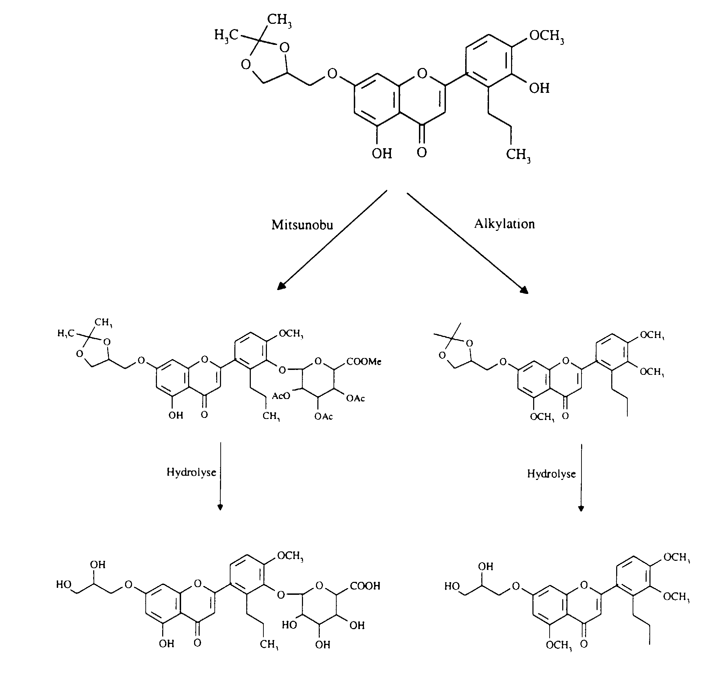

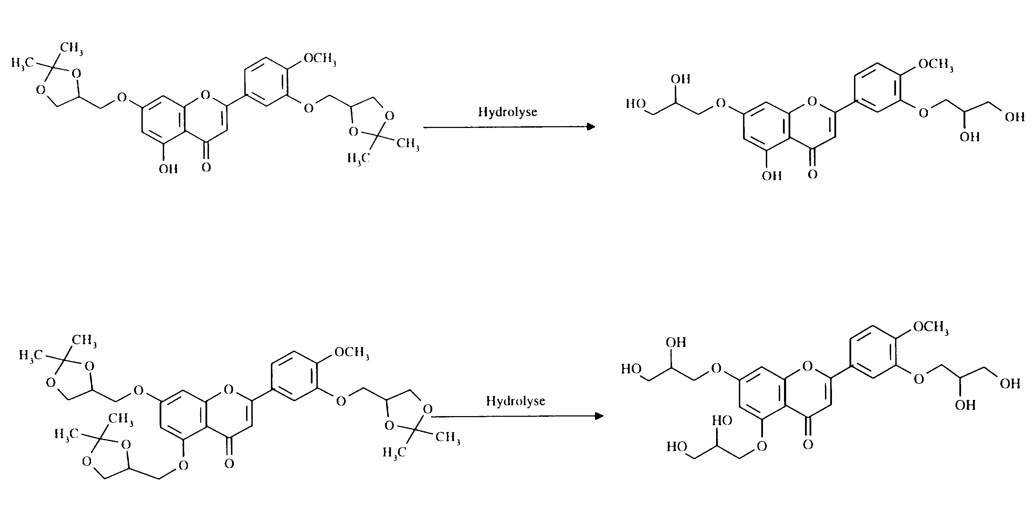

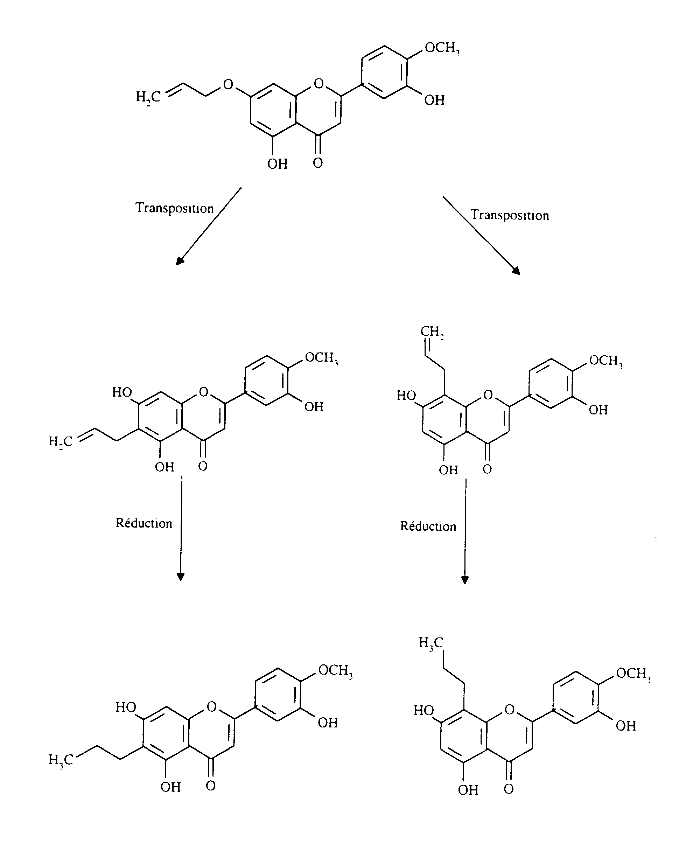

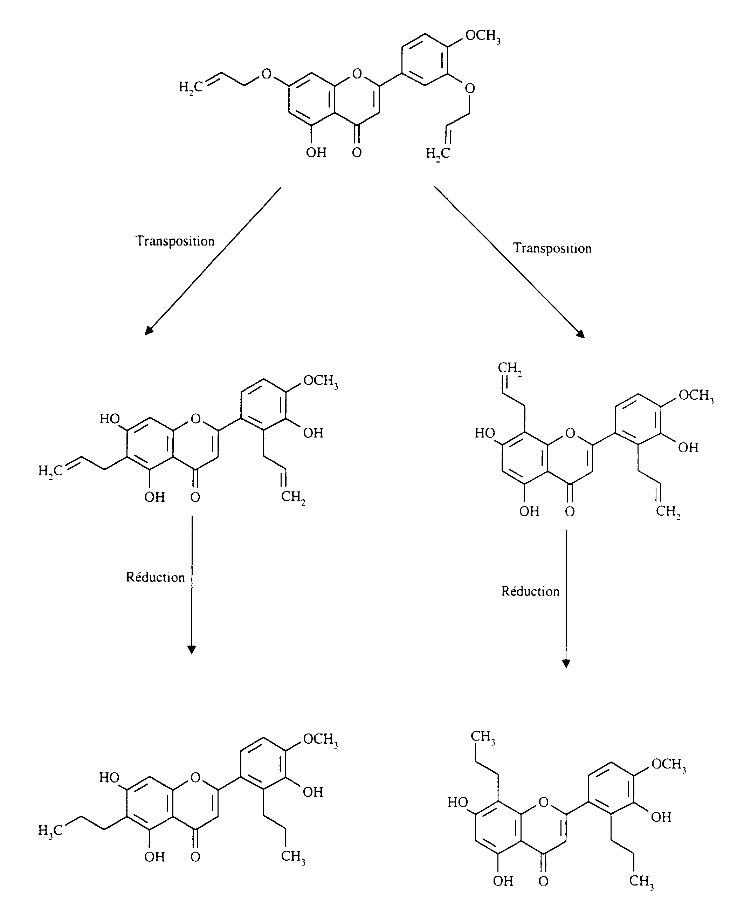

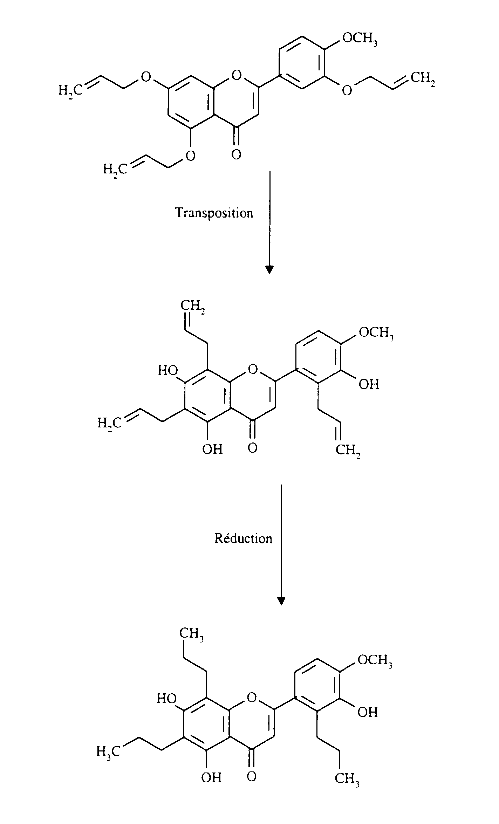

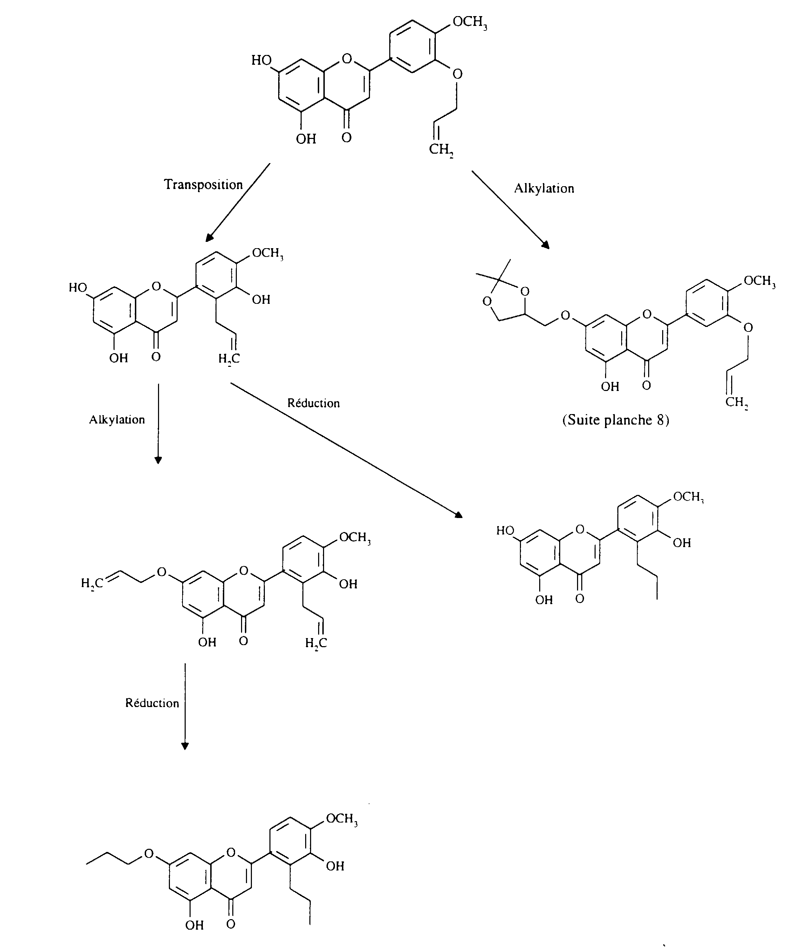

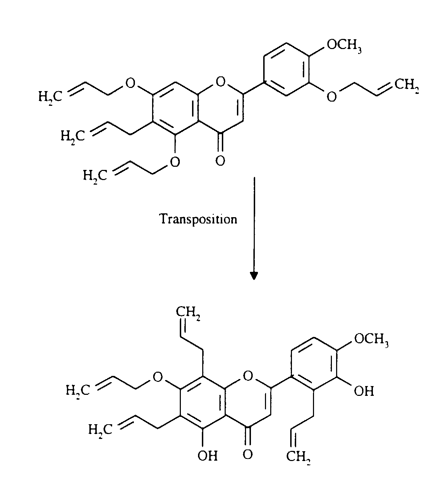

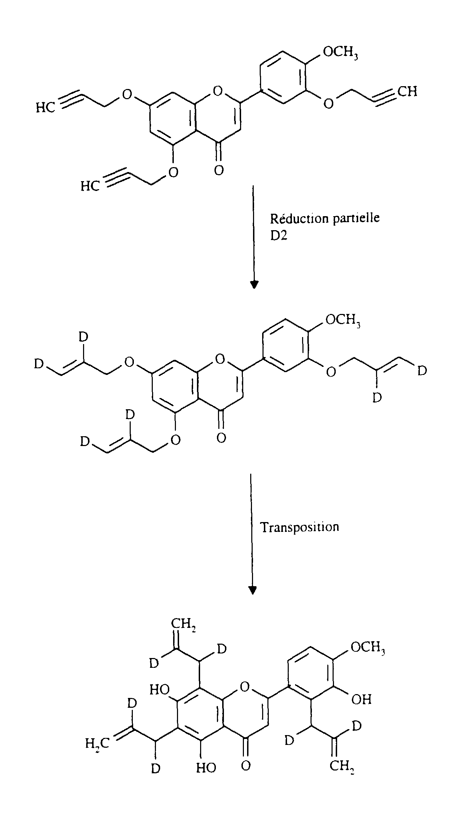

Les composés de formule I sont tous préparés à partir de la diosmétine, en utilisant des méthodes chimiques classiques mettant en jeu, par exemple, selon les cas, des réactions d'alkylation, transposition, estérification, hydrogénation, hydrogénolyse et/ou hydrolyse, comme illustré dans les planches 1 à 16.

La présente invention a également pour objet le procédé de préparation des composés de formule I caractérisé en ce que la diosmétine de formule II : 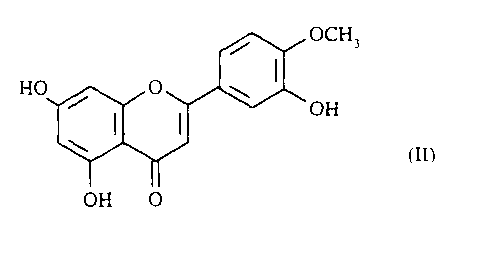 est traitée :
A/ soit par un composé de formule III : 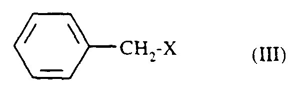 dans laquelle X représente un atome d'halogène tel que, par exemple, un atome de chlore ou de brome, ou un radical tosyloxy,
   pour obtenir un composé de formule IV : 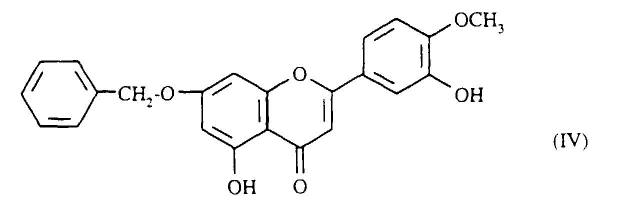 lequel est traité :
   a) soit par un halogénure d'allyle de formule H₂C=CH-CH₂-Hal dans laquelle Hal représente un atome de chlore ou de brome,
      pour donner un composé de formule V : 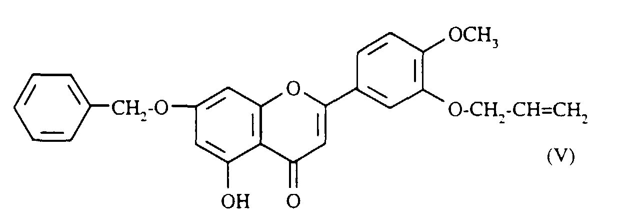 lequel par transposition donne le composé de formule VI : 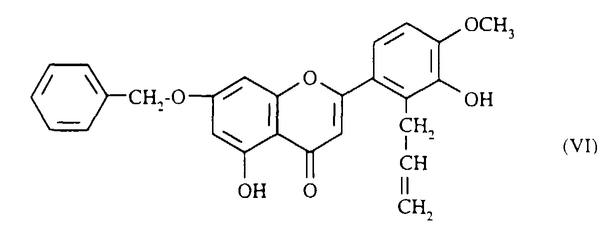 qui, par hydrogénation, donne le composé de formule Ia : 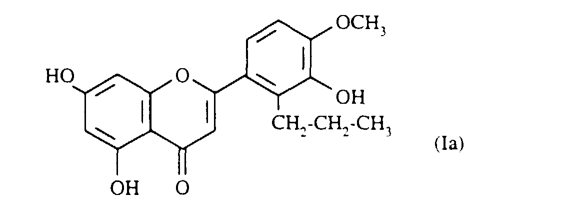 qui, traité à son tour par un halogénure d'allyle de formule H₂C=CH-CH₂-Hal dans laquelle Hal représente un atome de chlore ou de brome, conduit au composé de formule (Ib) : 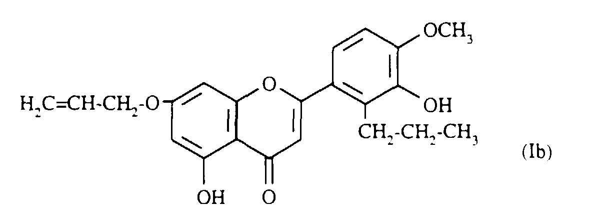 lequel par hydrogénation donne la composé de formule Ic : 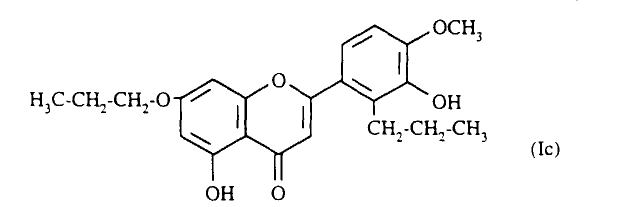
   b) soit par le tosylate de (2,2-diméthyl-1,3-dioxol-4-yl) méthyle de formule : 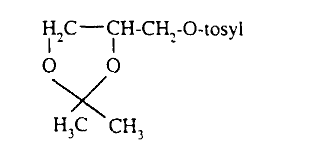 pour donner le composé de formule VII : 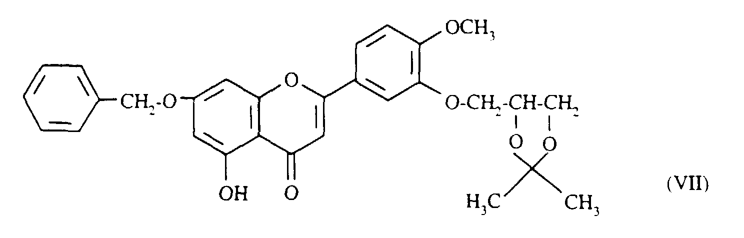 lequel par hydrogénation donne le composé de formule Id : 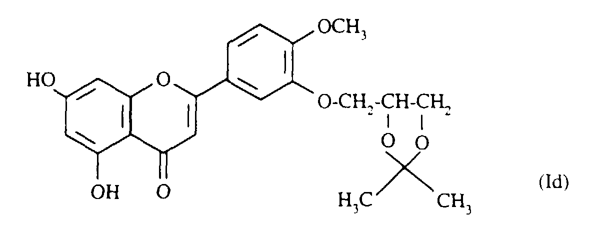 qui, traité par acide acétique et H₂O donne le composé de formule Ie : 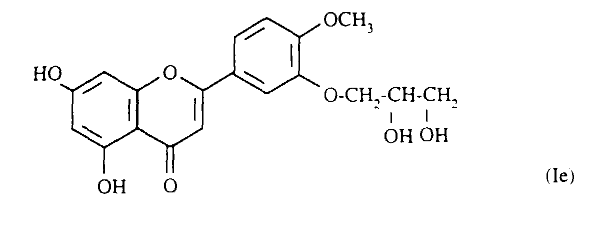 ou
B/ soit par un composé de formule III' :

   R'₂-X (III')

   dans laquelle :
   - R'₂ représente un radical allyle ou (2,2-diméthyl-1,3-dioxol-4-yl) méthyle ou propargyle, et
   - X a la signification précédemment définie pour obtenir, selon la quantité du composé III' par rapport à celle du composé II, l'un ou l'autre des composés de formule If, Ig et Ih : 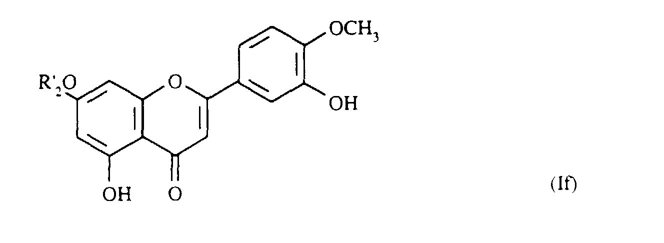 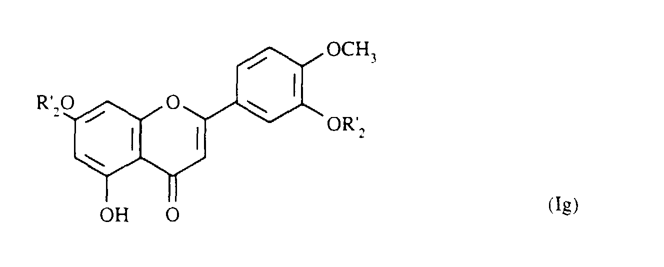 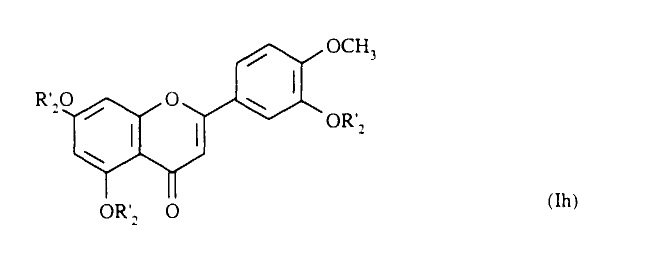 lesquels composés peuvent être soumis à des réactions ultérieures choisies parmi les réactions d'alkylation, transposition, estérification, réduction par l'hydrogène ou le deutérium et hydrolyse (comme illustré dans les planches ci-jointes) pour donner les produits de formule I autre que ceux de formule Ia à Ih dont la préparation est précédemment décrite.

L'ensemble des composés ainsi préparés forme l'ensemble des composés de formule I. Les exemples suivants illustrent la présente invention.

### Exemple 1

### 7-allyloxy-5-hydroxy-2-(3-hydroxy-4-méthoxy phényl)-4H-1-benzopyran-4-one.

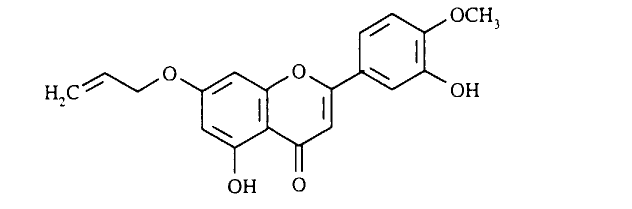

A 30 g de diosmétine mis dans 200 ml de diméthylformamide anhydre, portés à 80 °C, on ajoute, sous agitation, 10 g de bicarbonate de potassium puis goutte à goutte 11 g de bromure d'allyle. On maintient l'agitation à 80 °C pendant 8 heures, puis on laisse l'ensemble revenir à la température ambiante et filtre le milieu réactionnel.
Le résidu obtenu est recristallisé dans l'isopropanol. On obtient ainsi 18,5 g du composé attendu pur à 95 % environ.
Un échantillon analytique a été obtenu par chromatographie sur silice en éluant avec un mélange dichlorométhane/méthanol (98/2) ; PF: 174 °C.

### Exemple 2

### 7-allyloxy-5-hydroxy-2-(3-allyloxy-4-méthoxy phényl)-4H-1-benzopyran-4-one.

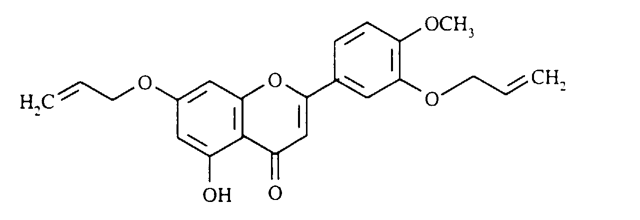

A 30 g de diosmétine, mis dans 200 ml de diméthylformamide et portés à 80 °C, on ajoute 20 g de bicarbonate de potassium, puis, goutte à goutte, 21 g de bromure d'allyle. Chauffage et agitation sont maintenus pendant 12 heures après la fin de l'addition, puis on laisse l'ensemble revenir à la température ambiante et filtre le milieu réactionnel. Après quoi, le solvant est distillé sous pression réduite et le résidu recristallisé dans l'isopropanol. On obtient ainsi 30 g du composé attendu, PF: 122 °C.

### Exemple 3

### 5,7-diallyloxy-2-(3-allyloxy-4-méthoxy phényl)-4H-1-benzopyran-4-one.

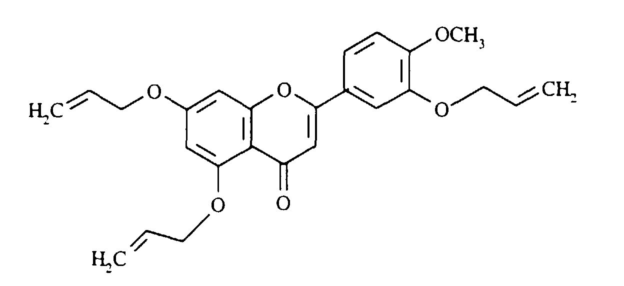

30 g de diosmétine, dans 300 ml de diméthylformamide sont ajoutés à une suspension de 11,7 g d'hydrure de sodium dans 150 ml de diméthylformamide. Le mélange est maintenu sous agitation à 40 °C jusqu'à fin du dégagement gazeux. On l'additionne alors de 48 g de bromure d'allyle et maintient l'agitation à 40 °C pendant 22 heures.
Les solvants et produits volatils sont distillés sous pression réduite. Le résidu est repris dans le chloroforme pendant 2 heures à 20 °C. On filtre alors le bromure de sodium. On amène à sec la solution de chloroforme et recristallise le résidu dans l'isopropanol. On obtient 29,4 g du composé attendu, PF: : 125 °C.

### Exemple 4

### (R,S) 5-hydroxy-7-[(2,2-diméthyl-1,3-dioxol-4-yl) méthoxy]-2-(3-hydroxy-4 méthoxyphényl)-4H-1-benzopyran-4-one.

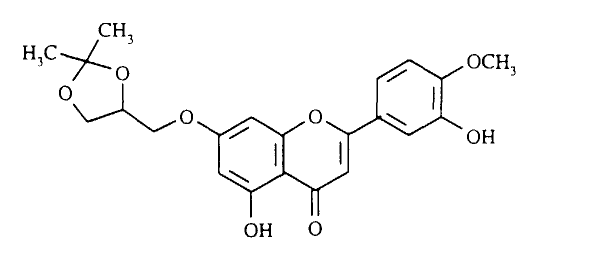

A 30 g de diosmétine et 11,5 g de bicarbonate de potassium dans 200 ml de diméthylformamide, portés à 90 °c, on ajoute goutte à goutte et sous agitation, 31,5 g de (R,S) p-toluènesulfonate de (2,2-diméthyl-1,3-dioxol-4 yl) méthyle. Chauffage et agitation sont ensuite maintenus pendant 24 heures, puis le milieu réactionnel est filtré. Le filtrat est concentré à sec par distillation du diméthylformamide sous pression réduite et le résidu est repris au dichlorométhane. La solution ainsi obtenue est amenée à sec et le résidu est recristallisé deux fois dans l'isopropanol. On obtient ainsi 22 g du composé attendu, PF : 180 °C.

### Exemples 5 et 6

En opérant comme décrit dans l'exemple 4, ont été préparés les composés objet des exemples suivants :
5) (R) 5-hydroxy-7-[(2,2-diméthyl-1,3-dioxol-4-yl) méthoxy]-2-(3-hydroxy-4-méthoxy phényl)-4H-1-benzopyran-4-one, PF : 142 °C, à partir du p-toluènesulfonate de (2,2-diméthyl-1,3-dioxol-4-yl) méthyle, sous forme d'isomère S.
6) (S) 5-hydroxy-7-[(2,2-diméthyl-1,3-dioxol-4-yl) méthoxy]-2-(3-hydroxy-4-méthoxy phényl)-4H-1-benzopyran-4-one, PF : 142 °C, à partir du p-toluènesulfonate de (2,2-diméthyl-1,3-dioxol-4-yl) méthyle, sous forme d'isomère R.

### Exemple 7

### 5-hydroxy-7-[(2,2-diméthyl-1,3-dioxol-4-yl)méthoxy]-2-{3-[(2,2-diméthyl-1,3-dioxol-4-yl) méthoxy]-4-méthoxy phényl}-4H-1-benzopyran-4-one.

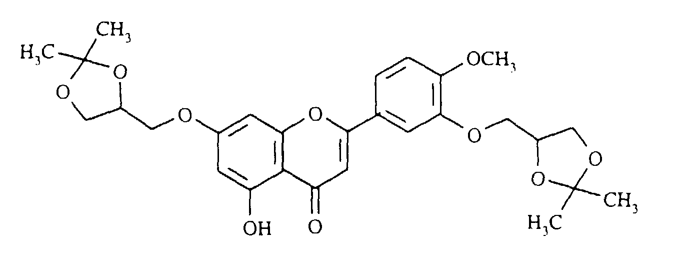

On chauffe à 80 °C, sous agitation pendant 30 heures, 30 g de diosmétine, 22 g de bicarbonate de potassium et 56 g de p-toluènesulfonate de (2,2-diméthyl-1,3-dioxol-4-yl) méthyle dans 500 ml de diméthylformamide additionnés de 25 ml d'hexaméthylphosphoramide. On distille ensuite sous pression réduite les solvants et produits volatils puis reprend le résidu par 500 ml de dichlorométhane. Le sel qui cristallise est éliminé par filtration, la phase organique est amenée à sec par distillation et le résidu est recristallisé dans l'isopropanol anhydre. On obtient ainsi 25,2 g de produit, pur à 95 % et utilisable tel quel.
Un échantillon pur du produit attendu a été préparé par chromatographie sur silice, en éluant avec un mélange CH₂Cl₂/CH₃OH progressivement enrichi jusqu'à 5 % de CH₃OH, PF : 155-156 °C.

### Exemple 8

### 5,7-di-[(2,2-diméthyl-1,3-dioxol-4-yl)méthoxy]-2-{3-[(2,2-diméthyl-1,3-dioxol-4-yl)méthoxy] -4-méthoxy phényl}-4H-1-benzopyran-4-one.

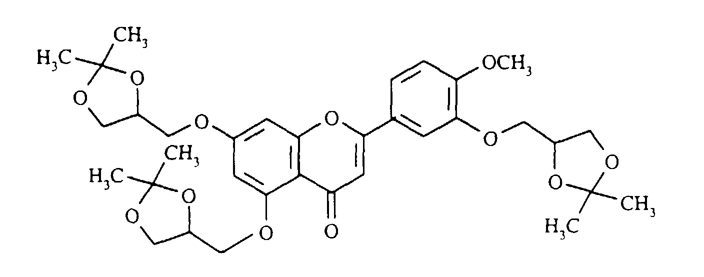

30 g de diosmétine et 10 g d'hydrure de sodium sont agités à température ambiante dans 100 ml de diméthylformamide anhydre. A la fin du dégagement gazeux, on ajoute lentement au mélange réactionnel 100 g de p-toluènesulfonate de (2,2-diméthyl-1,3-dioxol-4-yl) méthyle dissous dans 200 ml de diméthylformamide. L'ensemble est ensuite porté à 80 °C, sous agitation pendant 72 heures. Après quoi, on élimine le solvant par distillation sous pression réduite, et reprend le résidu par un mélange : dichlorométhane, méthanol et éther éthylique. Après filtration des parties insolubles, on amène le filtrat à sec et recristallise le résidu dans l'isopropanol. On obtient ainsi 22 g d'un composé fluorescent, pur à 94 % et utilisable tel quel. Un échantillon analytique du produit attendu a été obtenu par chromatographie sur silice, en éluant avec un mélange CH₂Cl₂/CH₃OH progressivement enrichi jusqu'à 5 % de CH₃OH.

### Exemple 9

### 5,7-dihydroxy-2-{3-[(2,2-diméthyl-1,3-dioxol-4-yl) méthoxy]-4-méthoxy phényl}-4H-1-benzopyran-4-one.

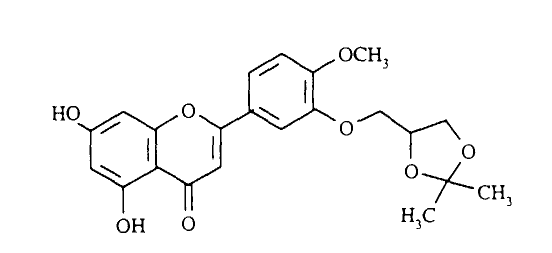

### A) Etape A

### 7-benzyloxy-5-hydroxy-2-{3-[(2,2-diméthyl-1,3-dioxol-4-yl) méthoxy]-4-méthoxy phényl}-4H-1-benzopyran-4-one.

39 g de 0-7 benzyl diosmétine et 10 g de bicarbonate de potassium, dans 300 ml de diméthyl formamide sont portés à 90 °C sous agitation et maintenu ainsi pendant 2 heures. Après quoi, on ajuste la température à 80 °c et ajoute goutte à goutte à ce mélange une solution de 31 g de p-toluène sulfonate de (2,2-diméthyl-1,3-dioxol-4-yl) méthyle dans 150 ml de diméthylformamide. Le mélange est maintenu sous agitation à 80 °C pendant 24 heures, puis le solvant est distillé sous pression réduite et le résidu repris dans le dichlorométhane. On filtre alors les parties insolubles, ramène le filtrat à sec et recristallise le résidu dans l'isopropanol. On obtient ainsi 45 g du composé attendu pur à 95 % et utilisable tel quel.

### B) Etape B

### 5,7-dihydroxy-2-{3-[(2,2-diméthyl-1,3-dioxol-4-yl)méthoxy]-4-méthoxy phényl}-4 H-1-benzopyran-4-one.

Les 45 g du composé obtenu à l'étape A sont mis dans 300 ml de diméthylformamide et soumis à une hydrogénolyse catalytique, sous une pression de 6300 hPa en présence de 300 mg de charbon palladié contenant 5 % de palladium.

Lorsque la quantité théorique d'hydrogène est absorbée, le milieu réactionnel est filtré, les solvants sont éliminés par distillation et le résidu obtenu, soit 36 g de composé attendu, est utilisé tel quel.

### Exemple 10

### 5,7-dihydroxy-2-[3-(2,3-dihydroxy propyloxy)-4-méthoxy phényl]-4H-1-benzopyran-4-one.

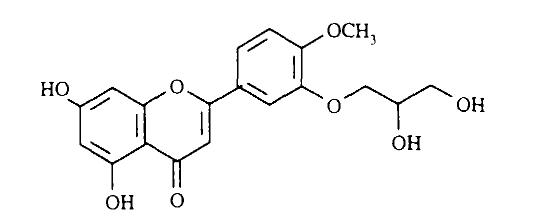

Les 36 g du composé obtenu dans l'exemple 9 sont ajoutés à 200 ml d'un mélange eau/acide acétique (1/1) et l'ensemble est porté à reflux jusqu'à ce que le mélange devienne homogène ; puis le reflux est maintenu pendant 15 minutes supplémentaires. La disparition de l'acétonide est vérifiée par chromatographie en couche mince, puis le milieu est refroidi à 0 °C, et le précipité formé est filtré puis séché. On obtient ainsi 30 g du composé attendu pur à 98 %.

Une recristallisation de ce composé dans un mélange isopropanol/eau conduit au produit pur, PF : 215-218 °C.

### Exemple 11

### 5.7-dihydroxy-2-(3-hydroxy-4-méthoxy-2-propyl phényl)-4H-1-benzopyran-4-one.

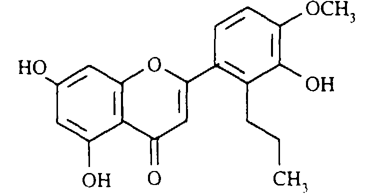

### A) Etape A

### 7-benzyloxy-5 hydroxy-2-(3-allyloxy-4 méthoxy phényl)-4H-1-benzopyran-4-one.

39 g de 0-7 benzyl diosmétine et 11 g de bicarbonate de potassium dans 300 ml de diméthylformamide sont portés puis maintenus à 90 °C sous agitation pendant deux heures. On laisse ensuite le mélange réactionnel revenir à température ambiante puis on l'additionne lentement de 16 g de bromure d'allyle en solution dans 100 ml de diméthylformamide. L'ensemble est porté à 60 °C et maintenu à cette température pendant 36 heures. Les parties volatiles sont ensuite distillées sous pression réduite et le résidu est repris au dichlorométhane. Les sels insolubles sont ensuite filtrés et le filtrat est concentré à sec. On obtient ainsi 40 g du composé attendu utilisable tel quel. Un échantillon analytique a été préparé par recristallisation dans l'isopropanol, PF : 219 °C.

### B) Etape B

### 7-benzyloxy-5-hydroxy-2-(2-allyl-3-hydroxy-4-méthoxy phényl)-4H-1-benzopyran-4-one.

Les 40 g du composé de l'étape A sont ajoutés à 300 ml de trichlorobenzène. L'ensemble est porté à reflux pendant 1 heure 30, puis refroidi à température ambiante et additionné d'éther de pétrole jusqu'à précipitation complète. L'huile obtenue se concrétise après quelques heures d'agitation. Les cristaux sont recueillis par filtration et lavés à l'éther de pétrole. On obtient ainsi 38 g du composé attendu que l'on utilise tel quel. Un échantillon analytique a été préparé par recristallisation dans l'isopropanol, PF : 234 °C.

### C) Etape C

### 5,7-dihydroxy-2-(3-hydroxy-4-méthoxy-2-propyl phényl)-4H-1-benzopyran4-one.

Les 38 g du composé final obtenu à l'étape B mis dans 350 ml de diméthylformamide sont soumis à une pression d'hydrogène de 6300 hPa, en présence de 300 mg de charbon palladié à 5 % de palladium comme catalyseur. Lorsque la quantité théorique d'hydrogène est absorbée, on filtre le milieu réactionnel, distille le filtrat sous pression réduite et recristallise le résidu dans l'isopropanol. On obtient ainsi 25 g du produit attendu, PF : 200 °C.

### Exemple 12

### 5-hydroxy-7-[(2,2-diméthyl-1,3-dioxol-4-yl)méthoxy]-2-(3,4-diméthoxyphényl)-4H-1-benzopyran-4-one.

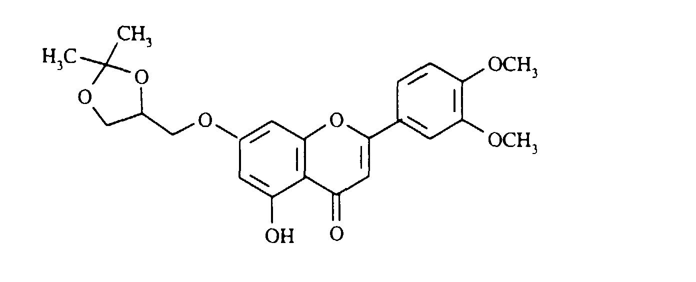

41 g de (R,S) 5-hydroxy-7-[(2,2-diméthyl-1,3-dioxol-4-yl)méthoxy]-2-(3-hydroxy-4-méthoxy phényl)-4H-1-benzopyran-4 one, préparé selon l'exemple 4, sont agités dans 800 ml de diméthylformamide avec 20 g de bicarbonate de potassium, à une température de 100 °C jusqu'à fin du dégagement gazeux. Le mélange est ensuite refroidi à 40 °C puis additionné, goutte à goutte, de 20,5 g d'iodure de méthyle. Lorsque l'addition est terminée, l'agitation est maintenue pendant une heure. Puis on distille le solvant et les produits volatils sous pression réduite. Le résidu est repris dans le minimum de dichlorométhane et la solution est filtrée sur 400 g de silice en éluant avec un mélange CH₂Cl₂/CH₃OH, progressivement enrichi, jusqu'à 5 % de CH₃OH. Après évaporation du solvant, on obtient 30 g du composé attendu, utilisable tel quel. Un échantillon analytique a été préparé par recristallisation dans l'isopropanol, PF : 166 °C.

### Exemple 13

### 5-hydroxy-2-(3,4-diméthoxy phényl)-7-(2,3-dihydroxy propyloxy)-4H-1-benzopyran-4-one.

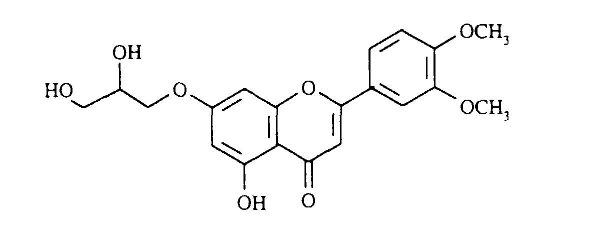

Les 30 g du composé préparé dans l'exemple 12 sont hydrolysés comme décrit dans l'exemple 10. Après une première recristallisation dans l'isopropanol puis une deuxième recristallisation dans l'acétonitrile, on obtient 22 g du composé attendu pur. PF : 149 °C.

### Exemple 14

### 5-méthoxy-7-[(2,2-diméthyl-1,3-dioxol-4-yl) méthoxy]-2-(3,4-diméthoxy phényl)-4H-1-benzopyran-4-one.

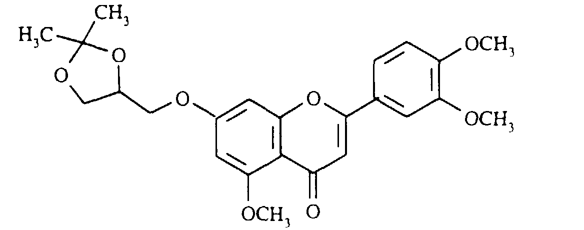

41 g de (R,S) 5-hydroxy-7-[(2,2-diméthyl dioxol-4-yl) méthoxy]-2-(3-hydroxy-4-méthoxy phényl)-4H-1-benzopyran-4-one, préparé selon l'exemple 4, sont agités dans 800 ml de diméthylformamide avec 40 g de carbonate de potassium, à une température de 100 °C, jusqu'à fin du dégagement gazeux. Le mélange est alors refroidi à 40°C puis additionné goutte à goutte de 40 g d'iodure de méthyle. On maintient ensuite agitation et température jusqu'à ce que le mélange cesse d'évoluer, puis effectue une chromatographie en couche mince. Après quoi, on distille le solvant et les produits volatils sous pression réduite et reprend le résidu par le minimum de dichlorométhane. La solution obtenue est filtrée sur 400 g de silice en éluant par un mélange CH₂Cl₂/CH₃OH, progressivement enrichi jusqu'à 5 % de CH₃OH.
Après évaporation du solvant, on obtient 27 g du composé attendu à l'état pur. PF : 93 °C.

### Exemple 15

### 5-méthoxy-2-(3,4-diméthoxy phényl)-7-(2,3-dihydroxy propyloxy)-4H-1-benzopyran-4-one.

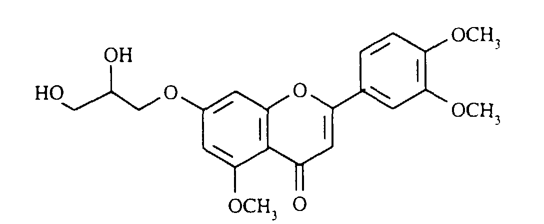

25 g du composé de l'exemple 14 sont hydrolysés, comme décrit dans l'exemple 10 pour conduire, après recristallisation dans l'acétonitrile à 18 g du composé attendu à l'état pur. PF : 143 °C.

### Exemple 16

### (R,S) 5-hydroxy-2-(3-hydroxy-4-méthoxy phényl)-7-(2,3-dihydroxy propyloxy)-4H-1-benzopyran-4-one.

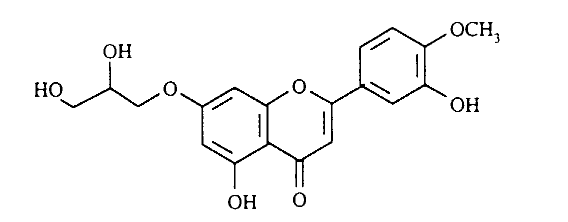

3 g de (R,S) 5-hydroxy-7-[(2,2-diméthyl-1,3-dioxol-4-yl) méthoxy]-2-(3-hydroxy-4-méthoxy phényl)4H-1-benzopyran-4-one, préparé selon l'exemple 4, sont hydrolysés comme décrit dans l'exemple 10 pour donner après recristallisation dans l'isopropanol 2,5 g du composé attendu pur, PF: 219-220 °C.

### Exemples 17 et 18

En opérant comme décrit dans l'exemple 16 ont été préparés les composés, objet des exemples suivants :
17) (R) 5-hydroxy-2-(3-hydroxy-4-méthoxy phényl)-7-(2,3-dihydroxy propyloxy)-4H-1-benzopyran-4-one, PF : 228 °C à partir du (S) 5-hydroxy-7-[(2,2-diméthyl-1,3-dioxol-4-yl) méthoxy]-2-(3-hydroxy4-méthoxy phényl)4H-1-benzopyran-4-one, préparé selon l'exemple 6.
   Pouvoir rotatoire [c] = 1 % dans DMSO à 20 °C:
   α(589 nm) = - 9° α(578 nm) = - 9° α(546 nm) = - 9,6°.
18) (S) 5-hydroxy-2-(3-hydroxy-4-méthoxy phényl)-7-(2,3-dihydroxy propyloxy)-4H-1-benzopyran-4-one, PF : 228 °C à partir du (R) 5-hydroxy-7-[(2,2-diméthyl-1,3-dioxol-4-yl) méthoxy]-2-(3-hydroxy-4-méthoxy phényl)-4H-1-benzopyran-4-one, préparé selon l'exemple 5.
   Pouvoir rotatoire [c] = 1 % dans DMSO à 20 °C:
   α(589 nm) = + 9° α(578 nm) = + 9° α(546 nm) = + 9,6°.

### Exemple 19

### 5,7-di-[(2,2-diméthyl-1,3-dioxol4-yl)méthoxy]-2-(3-hydroxy-4-méthoxy phényl)4H-1-benzopyran-4-one:

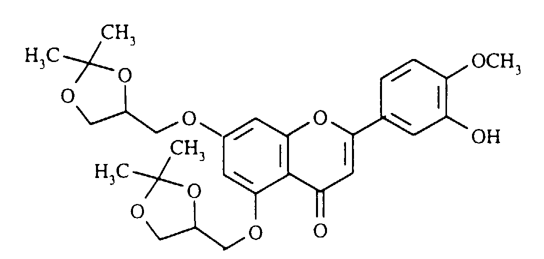

### A) Etape A

### 5-hydroxy-7-[(2,2-diméthyl dioxol-4-yl) méthoxy]-2-(3-benzyloxy-4-méthoxy phényl)-4H-1-benzopyran-4-one.

10 g de (R,S) 5-hydroxy-7-[(2,2-diméthyl dioxol4-yl) méthoxy]-2-(3-hydroxy-4-méthoxy phényl)-4H-1-benzopyran4-one préparés selon l'exemple 4, et 5,7 g de carbonate de potassium sont agités à reflux dans 300 il d'acétone, puis additionnés lentement de 5,8 g de bromure de benzyle dans 100 il d'acétone. Le mélange réactionnel est ensuite maintenu sous agitation à reflux pendant 12 heures, puis refroidi et filtré. L'acétone est éliminée du filtrat par distillation et le résidu obtenu qui est le 5-hydroxy-7-[(2,2-diméthyl dioxol-4-yl) méthoxy]-2-(3-benzyloxy-4-méthoxy phényl)4H-1-benzopyran-4-one pur à 98 % est utilisé tel quel. Un échantillon analytique a été préparé par recristallisation dans l'isopropanol, PF : 189-191 °C.

### B) Etape B

### 5,7-di-[(2,2-diméthyl-1,3-dioxol-4-yl)méthoxy]-2-(3-benzyloxy-4-méthoxy phényl)4H-1-benzopyran4-one.

8 g du composé préparé à l'étape A de l'exemple 19 sont alcoylés comme décrit dans l'exemple 4 avec du p-toluène sulfonate de (2,2-diméthyl-1,3-dioxol-4-yl) méthyle, en utilisant comme base de l'hydrure de sodium, dans le diméthylformamide. On obtient ainsi 4,5 g de produit attendu, sous forme de mélange de diastéréoisomères.

### C) Etape C

### 5,7-di-[(2,2-diméthyl-1,3-dioxol-4-yl)méthoxy]-2-(3-hydroxy-4-méthoxy phényl)-4H-1-benzopyran-4-one.

4 g du produit obtenu à l'étape B ci-dessus, sont soumis à une hydrogénolyse catalytique comme décrit dans l'exemple 13 pour conduire après traitement à environ 3,2 g du produit attendu à l'état pur, PF : 160 °C.

### Exemple 20

### 5,7-di-(2,3-dihydroxy propyloxy)-2-(3-hydroxy-4-méthoxy phényl)-4H-1-benzopyran-4-one.

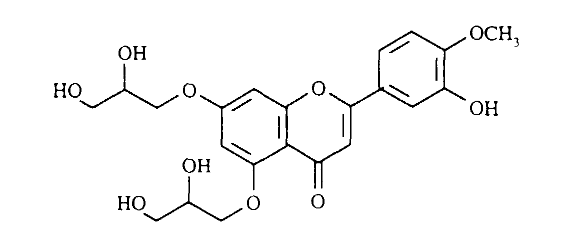

3 g du composé de l'exemple 19 sont hydrolysés dans les conditions énoncées dans l'exemple 16, puis traités par évaporation du milieu hydroacétique sous pression réduite. Le résidu est dissout dans le minimum de méthanol, filtré sur millipore et le filtrat est ramené à sec. Le résidu obtenu est traité par 50 ml d'eau bouillante puis l'ensemble est refroidi. Le précipité formé est alors agité à froid avec 50 ml d'éther éthylique pour donner 1,8 g du composé attendu à l'état pur, PF : 204 °C.

### Exemple 21

### 5-hydroxy-7-[(2,2-diméthyl-1,3-dioxol-4-yl) méthoxy]-2-(4-méthoxy-3-pivaloyloxy phényl)-4H-1-benzopyran-4-one.

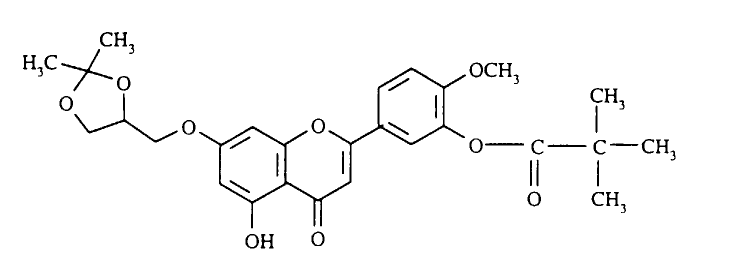

41 g de (R,S) 5-hydroxy-7-[(2,2-diméthyl-1,3-dioxol-4-yl)méthoxy]-2-(3-hydroxy-4-méthoxy phényl)-4H-1-benzopyran-4-one, préparé selon l'exemple 4, sont mis en suspension dans 1 250 ml de dioxane et 10 g de triéthylamine. L'ensemble est refroidi dans un bain de glace puis additionné goutte à goutte sous agitation de 12 g de chlorure de pivaloyle. Une fois d'addition terminée, on laisse revenir le mélange réactionnel à température ambiante, puis on le filtre. Le filtrat est concentré à sec et le résidu recristallisé dans l'acétonitrile. On obtient ainsi 48 g du composé attendu, PF : 204-205 °C.

### Exemple 22

### 5-hydroxy-7-[(2,2-diméthyl-1,3-dioxol-4-yl)méthoxy]-2-(3-isopropylcarbonyloxy-4-méthoxy phényl)-4H-1-benzopyran-4-one.

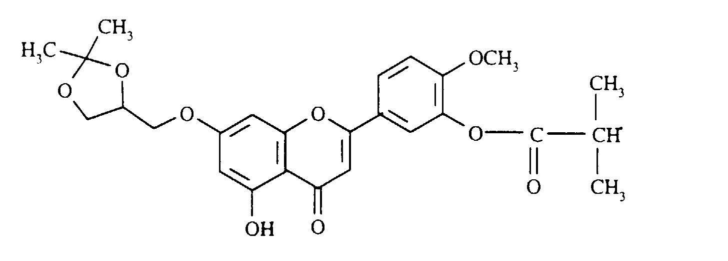

Ce composé a été préparé comme décrit dans l'exemple 21 mais en utilisant le chlorure d'isobutyryle à la place du chlorure de pivaloyle.

### Exemple 23

### 5-hydroxy-2-[(4-méthoxy-3-pivaloyloxy phényl)-7-(2,3-dihydroxy propyloxy)-4H-1-benzo pyran-4-one.

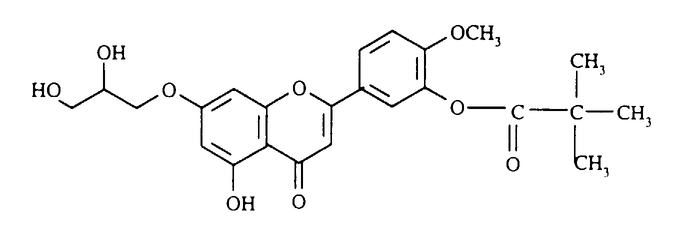

49,8 g du composé objet de l'exemple 21 sont chauffés progressivement dans un mélange de 300 il d'acide acétique et de 300 ml d'eau. Après addition, le mélange est maintenu à la même température pendant 5 minutes. Après addition de 300 ml d'eau, le milieu est ramené à température ambiante. Le précipité formé est recueilli par filtration puis recristallisé dans le mélange acétone/dichlorométhane. On obtient ainsi 35 g du composé attendu, PF : 130 °C.

### Exemple 24

### 5-hydroxy-2-(3-isopropyl carbonyloxy-4-méthoxy phényl)-7-(2,3-dihydroxy propyloxy)-4H-1-benzopyran-4-one.

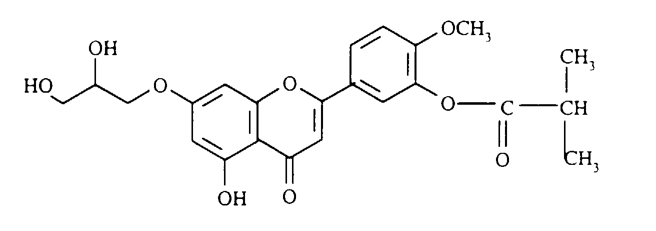

Ce composé, PF : 144-145 °C, a été préparé comme décrit dans l'exemple 23, à partir du composé objet de l'exemple 22.

### Exemple 25

### 5-allyloxy-2-(3-allyloxy-4-méthoxy phényl)-7-[(2,2-diméthyl-1,3-dioxol-4-yl)méthoxy]-4H-1-benzopyran-4-one.

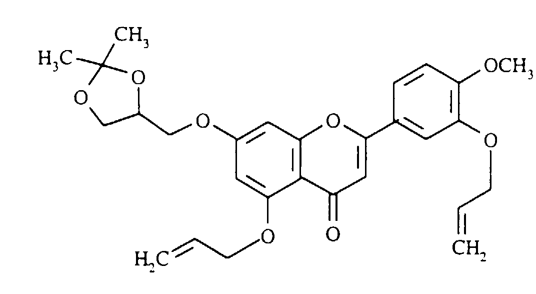

41 g du composé objet de l'exemple 4 sont alkylés avec 32 g de bromure d'allyle et 8 g d'hydrure de sodium, comme décrit dans l'exemple 3. On obtient ainsi, après recristallisation dans l'isopropanol, 32,8 g du composé attendu, pur à 95 %, utilisé tel quel. Un échantillon analytique a été préparé, par double recristallisation dans l'isopropanol , PF : 114 °C.

### Exemple 26

### 5-allyloxy-2-(3-allyloxy-4-méthoxy phényl)-7-[(2,3-dihydroxy propyloxy)-4H-1-benzopyran-4-one.

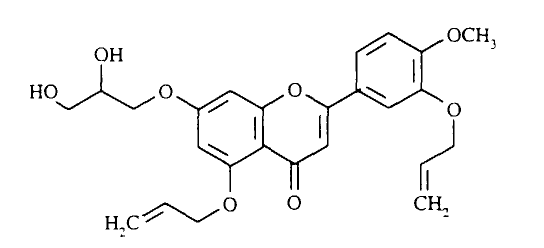

5 g du composé objet de l'exemple 25 sont hydrolysés comme décrit dans l'exemple 10 pour donner, après recristallisation dans l'isopropanol, 4,2 g du composé attendu, PF : 127 °C.

### Exemple 27 :

### 6-allyl-5-hydroxy-2-(2-allyl-3-hydroxy-4-méthoxy phényl)-7-[(2,2-diméthyl-1,3-dioxol-4-yl) méthoxy]-4H-1-benzopyran-4-one.

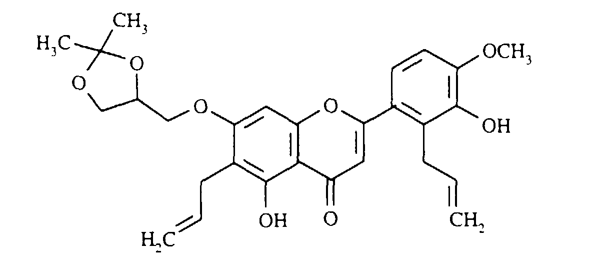

5 g du composé objet de l'exemple 25 sont traités comme décrit dans l'exemple 11 étape B pour donner 4,8 g du composé attendu utilisé tel quel.

### Exemple 28

### 6-allyl-5-hydroxy-2-(2-allyl-3-hydroxy-5-méthoxy phényl)-7-(2,3-dihydroxy propyloxy)-4H-1-benzopyran-4-one.

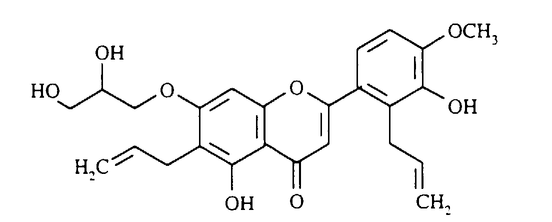

2,4 g de composé objet de l'exemple 27 sont hydrolysés pour donner, après recristallisation dans un mélange isopropanol/éther, 1,8 g du composé attendu, PF : 123°C.

### Exemple 29

### 5-hydroxy-7-[(2,2-diméthyl-1,3-dioxol-4-yl)méthoxy]-2-(3-hydroxy-4-méthoxy-2-propyl phényl)-6-propyl-4H-1-benzopyran-4-one.

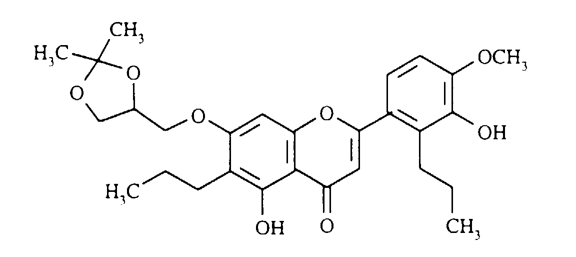

2,4 g de composé de l'exemple 27 sont hydrogénés dans 40 ml de diméthylformamide avec 100 mg de catalyseur (Palladium sur charbon à 5 %), sous une pression d'hydrogène de 6300 hPa. Après absorption de la quantité théorique d'hydrogène, le milieu est filtré sur millipore et le filtrat distillé sous pression réduite. Le résidu donne 2,4 g du produit attendu qui est utilisé tel quel. Un échantillon analytique a été préparé par recristallisation dans l'isopropanol, PF : 148 °C.

### Exemple 30

### 5-hydroxy-2-(3-hydroxy-4-méthoxy-2-propyl phényl)-6-propyl-7-(2,3-dihydroxy propyloxy)-4H-1-benzopyran-4-one.

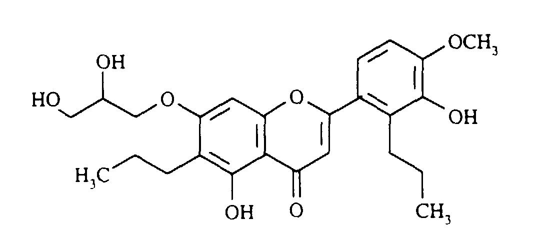

2,4 g du composé objet de l'exemple 29 sont hydrolysés, comme décrit dans l'exemple 10, pour donner, après recristallisation dans un mélange isopropanol/éther, 2 g du composé attendu, PF : 196 °C.

### Exemple 31

### 5-hydroxy-2-{[4-méthoxy-3-(6-carboxy-3,4,5-trihydroxy tétrahydropyran-2-yl)oxy] phényl}-7-(2,3-dihydroxypropyloxy)-4H-1-benzopyran-4-one.

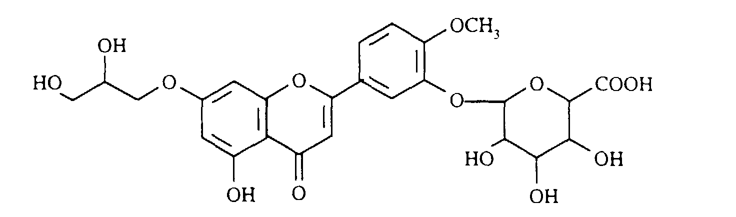

### A) Etape A

### 5-hydroxy-7-[(2,2-diméthyl-1,3-dioxol-4-yl)méthoxy]-2-{4-méthoxy-[3-(6-méthoxycarbonyl-3,4,5-triacétyloxy tétrahydropyran-2-yl)oxy]phényl}-4H-1-benzopyran-4-one.

Le couplage du composé objet de l'exemple 4 et du tri-0-acétyl glucuronate de méthyle est réalisé suivant la technique décrite par G.T. Badman et Coll., J. Organmet. Chem. 338 (1-2), 117-121 (1990). Ainsi, 410 mg du composé objet de l'exemple 4 sont agités pendant 48 heures avec 165 mg de diéthylazodicarboxylate et 500 mg de tri-0-acétyl glucuronate de méthyle dans 25 ml de tétrahydrofurane à température ambiante et sous atmosphère inerte. Après quoi, le solvant est éliminé sous pression réduite et le résidu chromatographié sur 30 g de silice en éluant avec un mélange cyclohexane/acétate d'éthyle (1/1). On a ainsi obtenu 390 mg du composé attendu sous forme d'huile.

### B) Etape B

### 5-hydroxy-2-{[4-méthoxy-3-(6-carboxy-3,4,5-trihydroxy tétrahydropyran-2-yl) oxy] phényl}-7-(2,3-dihydroxy propyloxy)-4H-1-benzopyran-4-one.

L'hydrolyse du composé obtenu à l'étape A est réalisée selon la technique décrite par Cheukk. Lav et Coll., J. Med. Chem., 35, (7), 1299-1318 (1992).
Ainsi, l'huile obtenue à l'étape A ci-dessus est additionnée à un mélange de 8 ml de soude normale, et 12 ml de méthanol. Le tout est agité à température ambiante pendant environ 4 heures, puis le mélange est traité par 8 ml d'HCl normal, et agité à température ambiante pendant 30 minutes. Après quoi, on y additionne 1 ml d'acide chlorhydrique normal et agite l'ensemble pendant 30 minutes à 25 °C sous atmosphère inerte. La solution est lyophilisée et le résidu chromatographié sur silice en éluant avec un mélange acétate d'éthyle/acide acétique progressivement enrichi en acide acétique. Après évaporation du solvant des fractions pures, on obtient 250 mg du composé attendu, sous forme d'une gomme amorphe.

### Exemple 32

### (R,S) 5-hydroxy-7-[(2,2-diméthyl-1,3-dioxol-4-yl)méthoxy]-2-(3-allyloxy-4-méthoxy phényl)-4H-1-benzopyran-4-one.

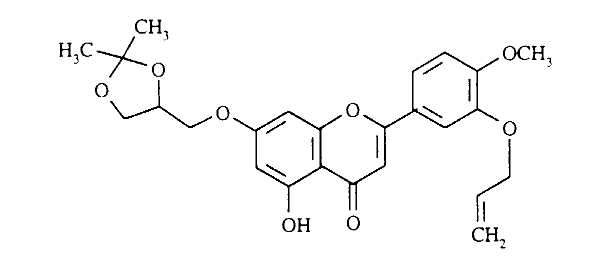

41 g du composé objet de l'exemple 4 et 15 g de carbonate de potassium sont agités dans 500 ml de diméthylformamide à 90°C, puis additionnés de 15 g de bromure d'allyle. Le chauffage est maintenu pendant encore 2 heures après l'addition ; puis le mélange est laissé revenir à température ambiante puis filtré. On concentre à sec le filtrat en distillant le diméthylformamide. Le résidu obtenu est repris par 1 000 ml de dichlorométhane. Après filtration, le filtrat est amené à sec par distillation du dichlorométhane, et le résidu est recristallisé dans l'isopropanol. On obtient ainsi 36 g du composé attendu, PF : 139 °C.

### Exemples 33 et 34

En opérant comme décrit dans l'exemple 32, ont été préparés les composés objet des deux exemples suivants :
33) (R) 5-hydroxy-7-[(2,2-diméthyl-1,3-dioxol-4-yl)méthoxy]-2-(3-allyloxy-4-méthoxy phényl)-4H-1-benzopyran4-one, à partir du composé objet de l'exemple 5.
34) (S) 5-hydroxy-7-[(2,2-diméthyl-1,3-dioxol-4-yl)méthoxy]-2-(3-allyloxy-4-méthoxy phényl)-4H-1-benzopyran-4-one, à partir du composé objet de l'exemple 6.

### Exemple 35

### 5-hydroxy-7-[(2,2-diméthyl-1,3-dioxol-4-yl)méthoxy]-2-(4-méthoxy-3-propyloxy phényl)-4H-1-benzopyran-4-one

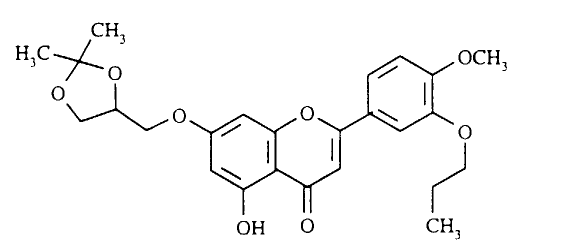

4,5 g du composé objet de l'exemple 32 ont été hydrogénés, comme décrit dans l'exemple 29, pour donner 4,5 g du composé attendu, utilisable tel quel.

### Exemple 36

### 5-hydroxy-2-(4-méthoxy-3-propyloxy phényl)-7-(2,3-dihydroxy propyloxy)-4H-1-benzopyran 4-one.

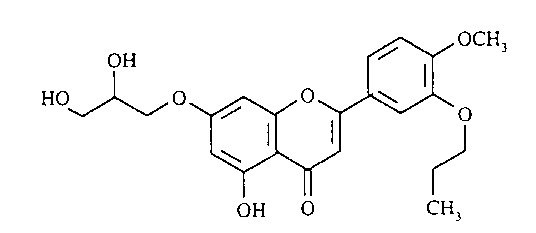

4,5 g du composé objet de l'exemple 35 ont été hydrolysés comme décrit dans l'exemple 10 pour donner, après recristallisation dans un mélange isopropanol/éther, 3,8 g du composé attendu, PF : 157 °C.

### Exemple 37

### (R,S) 5-hydroxy-7-[-2,2-diméthyl-1,3-dioxol-4-yl) méthoxy]-2-(2-allyl-3-hydroxy-4-méthoxy phényl)-4H-1-benzopyran4-one.

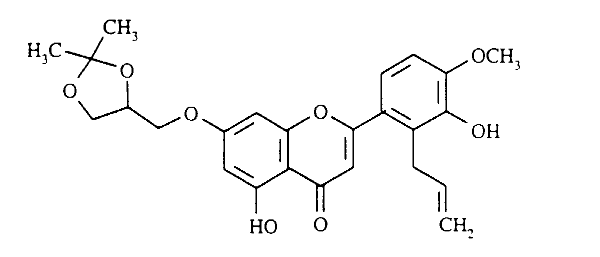

45,4 g du composé objet de l'exemple 32 ont été traités comme décrit dans l'exemple 11 étape B, puis recristallisés dans l'isopropanol, pour donner ainsi 43 g du composé attendu.

### Exemples 38-39

En opérant comme décrit dans l'exemple 37, ont été préparés les composés objet des deux exemples suivants :
38) (R) 5-hydroxy-7-[(2,2-diméthyl-1,3-dioxol-4-yl) méthoxy]-2-(2-allyl-3-hydroxy-4-méthoxy phényl)-4H-1-benzopyran-4-one, à partir du composé objet de l'exemple 33.
39) (S) 5-hydroxy-7-[(2,2-diméthyl-1,3-dioxol-4-yl) 1,3-dioxol-4-yl) méthoxy]-2-(2-allyl-3-hydroxy-4-méthoxy phényl)-4H-1-benzopyran-4-one, à partir du composé objet de l'exemple 34.

### Exemple 40

### (R,S) 5-hydroxy-2-(2-allyl-3-hydroxy-4-méthoxyphényl)-7-(2,3-dihydroxy propyloxy)-4H-1-benzopyran-4-one

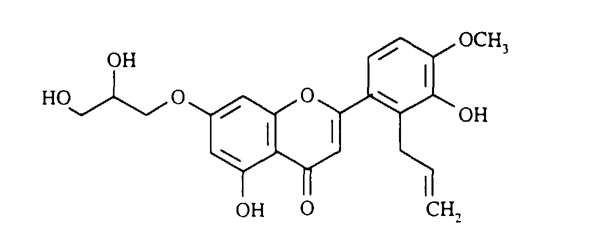

45,4 g du composé objet de l'exemple 37 ont été ajoutés à un mélange de 300 ml d'eau et 300 ml d'acide acétique.
L'ensemble a été chauffé progressivement jusqu'à dissolution complète, puis pendant encore 5 minutes après dissolution. Après addition de 200 ml d'eau et retour à température ambiante, le précipité obtenu a été filtré puis recristallisé dans l'isopropanol pour donner finalement 35,5 g du composé attendu, PF : 134 °C.

### Exemple 41-42

En opérant comme décrit dans l'exemple 40, ont été préparés les composés objet de deux exemples suivants :
41) (R) 5-hydroxy-2-(2-allyl-3-hydroxy4-méthoxyphényl)-7-(2,3-dihydroxy propyloxy)-4H-1-benzopyran-4-one, PF : 164 °C, à partir du composé objet de l'exemple 39.
   Pouvoir rotatoire [c] = 1 % dans le diméthylformamide :
   α(589 nm) = - 6,7° α(578 nm) = - 7,0° α(546 nm) = - 7,9°.
42) (S) 5-hydroxy-2-(2-allyl-3-hydroxy-4-méthoxyphényl)-7-(2,3-dihydroxy propyloxy)-4H-1-benzopyran-4-one, PF : 164 °C à partir du composé objet de l'exemple 38.
   Pouvoir rotatoire [c] = 1 % dans le diméthylformamide :
   α(589 nm) = + 6,7° α(578 nm) = + 7,0° α(546 nm) = + 7,9°.

### Exemple 43

### (R,S) 5-hydroxy-7-[(2,2-diméthyl-1,3-dioxol-4-yl) méthoxy]-2-(3-hydroxy-4-méthoxy-2-propyl phényl)-4H-1-benzopyran-4-one.

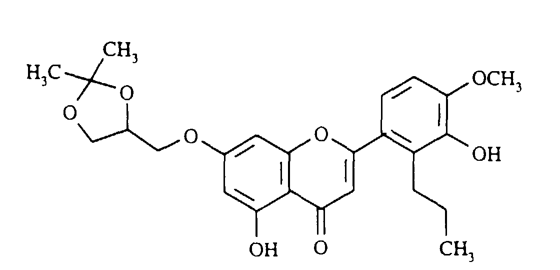

45,4 g du composé objet de l'exemple 37, sont dissous à chaud dans 800 ml d'éthanol à 96 %. Le mélange est hydrogéné sous une pression d'hydrogène de 6300 hPa en présence de 2 g de palladium sur charbon à 5 %. Après absorption de la quantité stoechiométrique d'hydrogène, le mélange réactionnel est filtré et le filtrat amené à sec par distillation de l'éthanol sous pression réduite, pour donner 45 g du composé attendu, PF : 121 °C.

### Exemples 44-45

En opérant comme décrit dans l'exemple 43, ont été préparés les composés objet des deux exemples suivants :
44) (R) 5-hydroxy-7-[(2,2-diméthyl-1,3-dioxol-4-yl) méthoxy]-2-(3-hydroxy-4-méthoxy-2-propylphényl)-4H-1-benzopyran-4-one, à partir du composé objet de l'exemple 38.
45) (S) 5-hydroxy-7-[(2,2-diméthyl-1,3-dioxol-4-yl) méthoxy]-2-(3-hydroxy-4-méthoxy-2-propyl phényl)-4H-1-benzopyran-4-one, à partir du composé objet de l'exemple 39.

### Exemple 46

### (R,S) 5-hydroxy-2-(3-hydroxy4-méthoxy-2-propyl phényl)-7-(2,3-dihydroxypropyloxy)-4H-1-benzopyran-4-one.

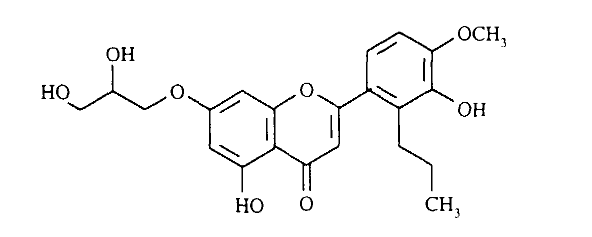

45,6 g du composé objet de l'exemple 43 sont ajoutés à un mélange de 300 ml d'eau et 300 ml d'acide acétique. L'ensemble est chauffé progressivement jusqu'à dissolution complète, après quoi le chauffage est maintenu pendant encore 5 minutes, puis on ajoute 200 ml d'eau.
Après retour à température ambiante, le précipité obtenu est filtré, lavé à l'eau puis recristallisé dans l'isopropanol, pour donner le composé racémique attendu qui existe sous des formes cristallines différentes :
- la forme α (PF : 145 °C, ν CO (IR) : 1672 cm⁻¹) obtenue en dissolvant 40 g du produit brut dans 600 ml d'isopropanol à ébullition puis en laissant revenir lentement à température ambiante et en laissant cristalliser pendant une semaine sans agiter ;
- la forme β (PF : 156 °C, ν CO (IR) : 1658 cm⁻¹) obtenue en dissolvant 40 g de produit brut dans 200 ml d'isopropanol à ébullition, puis en refroidissant rapidement en agitant ;
- la forme γ (PF : 121-3 °C,ν CO (IR) : 1668 cm⁻¹) obtenue en recristallisant 40 g du produit brut dans 400 ml de méthanol anhydre.

### Exemples 47-48

En opérant comme décrit dans l'exemple 46, ont été préparés les composés objet des deux exemples suivants :
47) (R) 5-hydroxy-2-(3-hydroxy-4-méthoxy-2-propyl phényl)-7-(2,3-dihydroxy propyloxy)-4H-1-benzopyran-4-one, PF : 159 °c, à partir du composé objet de l'exemple 45.
   Pouvoir rotatoire [c] = 0,5 % dans CH₃OH :
   α(589 nm) = - 7,1° α(578 nm) = - 7,6° α(546 nm) = - 8,7°.
48) (S) 5-hydroxy-2-(3-hydroxy-4-méthoxy-2-propyl phényl)-7-(2,3-dihydroxy propyloxy)-4H-1-benzopyran-4-one, PF : 159 °C,) partir du composé objet de l'exemple 44.
   Pouvoir rotatoire [c] = 0,5 % dans CH₃OH :
   α(589 nm) = + 7,1° α(578 nm) = +7,6° α(546 nm) = + 8,7°.

### Exemple 49

### (R,S) 5-hydroxy-2-(3-hydroxy-4-méthoxy-2-propyl phényl)-7-(3-acétyloxy-2-hydroxy propy loxy)-4H-1-benzopyran-4-one.

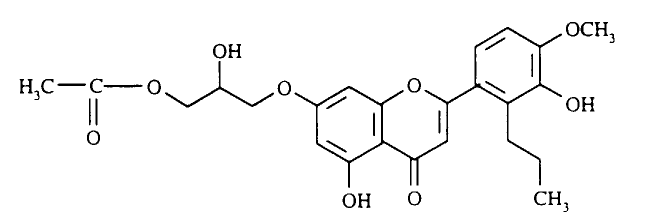

Ce composé racémique, PF : 147-148 °C, a été obtenu par estérification du composé objet de l'exemple 46.

### Exemples 50-51

Par analogie avec l'exemple 49, ont été préparés les composés objet des deux exemples suivants :
50) (R) 5-hydroxy-2-(3-hydroxy-4-méthoxy-2-propyl phényl)-7-(3-acétyloxy-2-hydroxy propy loxy)-4H-1-benzopyran-4-one, PF : 140 °C, à partir du composé objet de l'exemple 48.
51) (S) 5-hydroxy-2-(3-hydroxy-4-méthoxy-2-propylphényl)-7-(3-acétyloxy-2-hydroxy propyloxy)-4H-1-benzopyran-4-one, PF : 140 °C, à partir du composé objet de l'exemple 47.

### Exemple 52

### (R,S) 5- tert- butyl carbonyloxy -7- [(2,2-diméthyl-1,3-dioxol-4-yl) méthoxy]-2-(3-hydroxy-4-méthoxy-2-propyl phényl)4H-1-benzopyran-4-one.

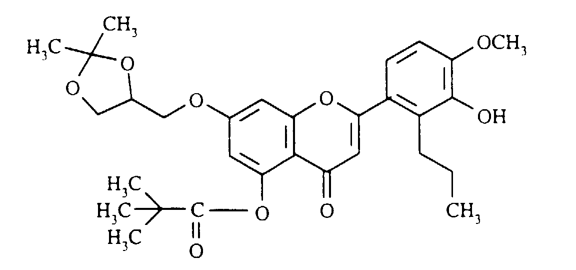

4,56 g du composé objet de l'exemple 43 sont dissous dans 100 ml de dioxane et 1 g de triéthylamine. La solution est refroidie sur un bain de glace puis additionnée goutte à goutte de 1,2 g de chlorure de pivaloyle. Après retour à température ambiante, puis chauffage à reflux sous agitation pendant 48 heures, on laisse le mélange revenir à température ambiante et filtre le précipité formé. Le filtrat est concentré à sec par distillation du dioxane sous pression réduite, et le résidu obtenu, est repris par 50 ml d'éther éthylique. Une agitation vigoureuse jusqu'à obtention d 'un précipité fin permet d'obtenir 2,7 g du composé attendu.

### Exemple 53

### (R,S) 5-tert-butyl carbonyloxy-2-(3-hydroxy-4-méthoxy-2-propyl phényl)-7-(2,3-dihydroxy propyloxy)-4H-1-benzopyran-4-one.

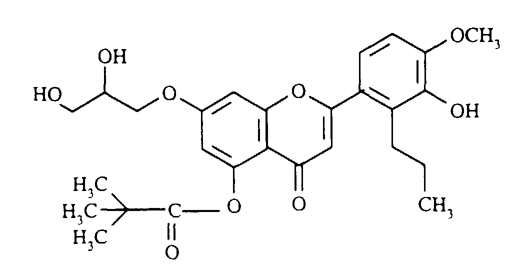

5,4 g du composé objet de l'exemple 52 sont ajoutés à un mélange de 30 ml d'eau et 30 ml d'acide acétique. L'ensemble est chauffé progressivement jusqu'à dissolution complète. Après retour à la température ambiante, on ajoute 20 ml d'eau, et recueille le précipité gommeux obtenu en éliminant le surnageant. Cette gomme est dissoute dans le dichlorométhane et la solution obtenue est séchée sur sulfate de magnésium. Le solvant est éliminé sous pression réduite et le résidu est recristallisé dans l'éther éthylique anhydre. On obtient ainsi 3,6 g du composé attendu, PF : 158 °C.

### Exemple 54

### 5-méthoxy-7-[(2,2-diméthyl-1,3-dioxol-4-yl) méthoxy]-2-(3-allyloxy-4-méthoxy phényl)-4H-1-benzopyran-4-one.

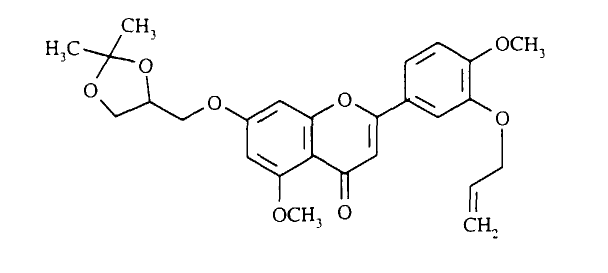

4,5 g du composé objet de l'exemple 32 sont méthylés dans les conditions décrites dans l'exemple 14, en utilisant 2 g de carbonate de potassium et 2 g d'iodure de méthyle, pour donner, après traitement, 3,7 g du composé attendu utilisable tel quel. Un échantillon analytique a été préparé par recristallisation dans l'acétate d'éthyle, PF : 156 °C.

### Exemple 55

### 5-méthoxy-7-[(2,2-diméthyl-1,3-dioxol-4-yl) méthoxy]-2-(2-allyl-3-hydroxy-4-méthoxy phényl)-4H-1-benzopyran-4-one.

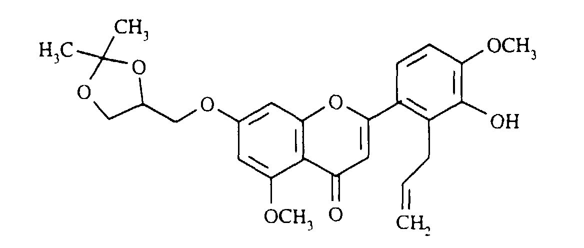

3,5 g du composé objet de l'exemple 54 sont transposés, comme décrit dans l'exemple 11 étape B, pour donner 3,5 g du composé attendu utilisable tel quel.

### Exemple 56

### 5-méthoxy-7-[(2,2-diméthyl-1,3-dioxol-4-yl) méthoxy]-2-(3-hydroxy4-méthoxy-2-propyl phényl)-4H-1-benzopyran4-one.

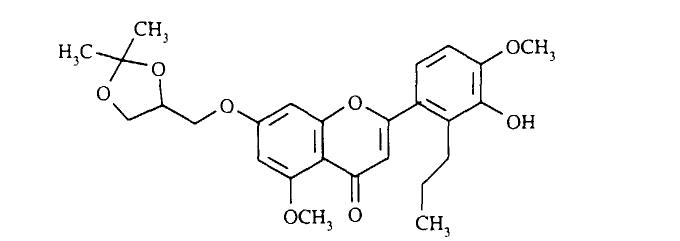

3,5 g du composé objet de l'exemple 55 sont hydrogénés dans les conditions décrites dans l'exemple 29 pour donner environ 3,5 g du composé attendu utilisable tel quel.

### Exemple 57

### 5-méthoxy-2-(3-hydroxy-4-méthoxy-2-propyl phényl)-7-(2,3-dihydroxy propyloxy)-4H-1-benzopyran-4-one.

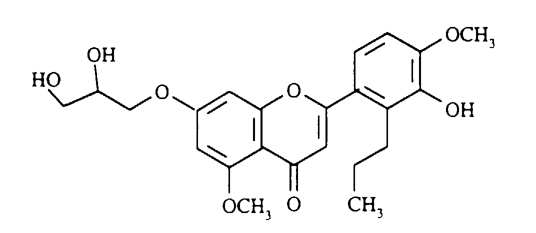

3,5 g du composé objet de l'exemple 56 sont hydrolysés comme décrit dans l'exemple 10 pour donner environ 2,7 g du composé attendu.

### Exemple 58

### 5-hydroxy-2-[4-méthoxy-2-propyl-3-(6-carboxy-3,4,5-trihydroxy tétrahydropyran-2-yl oxy) phényl]-7-(2,3-dihydroxy propyloxy)-4H-1-benzopyran4-one.

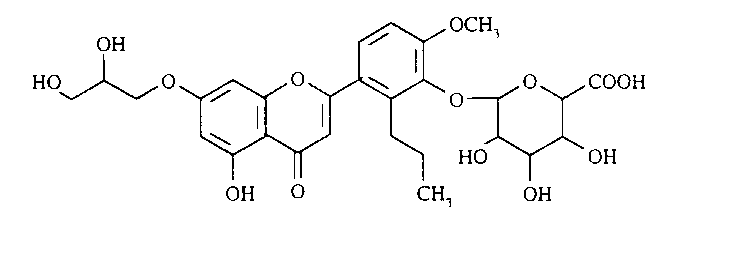

### A) Etape A

### 5-méthoxy-7-[(2,2-diméthyl-1,3-dioxol-4-yl) méthoxy]-2-[4-méthoxy-2-propyl-3-(6-méthoxy carbonyl-3,4,5-triacétyloxy tétrahydropyran-2-yl oxy) phényl]-4H-1-benzopyran-4-one.

Ce composé a été obtenu à partir du composé objet de l'exemple 43, en opérant comme décrit dans l'exemple 31 étape A.

### B) Etape B

### 5-hydroxy-2-[4-méthoxy-2-propyl-3-(6-carboxy-3,4,5-trihydroxy tétrahydropyran-2-yl oxy) phényl]-7-(2,3-dihydroxy propyloxy)-4H-1-benzopyran-4-one.

Ce composé amorphe a été obtenu à partir du composé ci-dessus préparé à l'étape A, en opérant comme décrit dans l'exemple 31 étape B.

### Exemple 59

### 5-hydroxy-2-[4-méthoxy-3-(2,3-dihydroxy propyloxy) phényl]-7-(2,3-dihydroxy propyloxy)-4H-1-benzopyran-4-one.

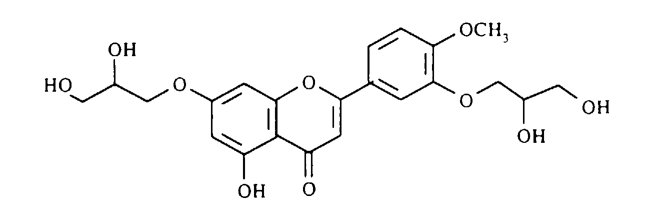

1g du composé objet de l'exemple 7 est hydrolysé dans les conditions décrites dans l'exemple 16 pour donner, après recristallisation dans l'isopropanol, 0,6 g du composé attendu, PF 166 °C.

### Exemple 60

### 2-[4-méthoxy-3-(2,3-dihydroxy propyloxy) phényl]-5,7-(2,3-dihydroxy propyloxy)-4H-1-benzopyran-4-one.

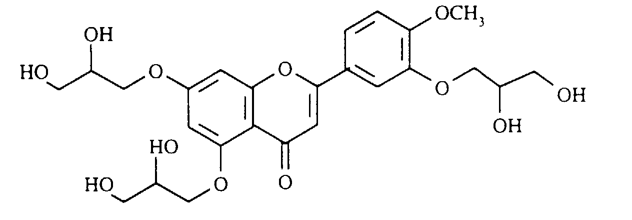

3g du composé objet de l'exemple 8 sont traités dans les conditions décrites dans l'exemple 20 à la différence que le résidu n'a pas été repris dans l'eau bouillante. Après recristallisation dans l'éther, on a obtenu environ 1,4 g du composé attendu, PF :117-120 °C.

### Exemple 61

### 6-allyl-5,7-dihydroxy-2-(3-hydroxy-4-méthoxy phényl)-4H-1-benzopyran-4-one.

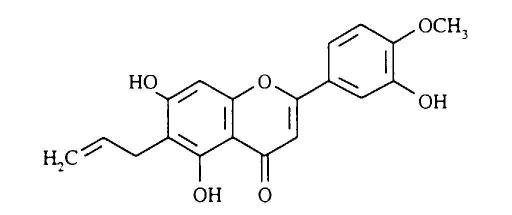

7 g du composé objet de l'exemple 1 sont transposés dans les conditions décrites dans l'exemple 11 étape B. Le mélange obtenu après élimination du solvant est chromatographié sur silice en éluant avec un mélange CH₂Cl₂/CH₃COOC₂H₅ (90/10). On a ainsi obtenu après réunion des fractions identiques et distillation des solvants :
2,6 g du composé attendu objet du présent exemple et 4 g du composé objet de l'exemple 62 ci-après désigné, chacun de ces composés étant utilisables tels quels. Par recristallisation de chacun d'eux dans l'isopropanol ont été obtenus des échantillons analytiques à l'état pur, des composés objet :
- de l'exemple 61, PF : 238 °C, et
- de l'exemple 62, PF : 255 °C.

### Exemple 62

### 8-allyl-5,7-dihydroxy-2-(3-hydroxy-4-méthoxy phényl)-4H-1-benzopyran-4-one.

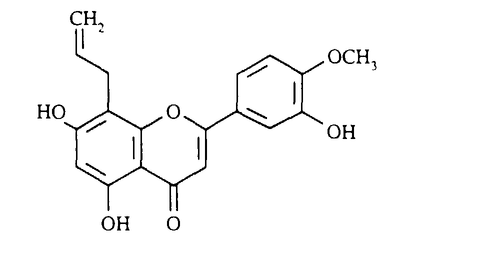

Ce composé a été préparé comme décrit dans l'exemple 61 ci-dessus.

### Exemple 63

### 5,7-dihydroxy-2-(3-hydroxy-4-méthoxy phényl)-6-propyl-4H-1-benzopyran-4-one.

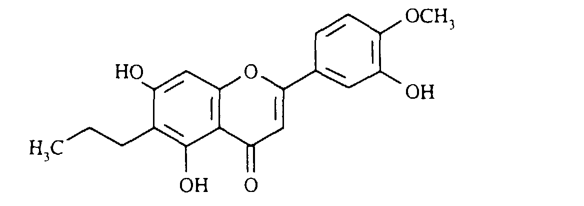

2,5 g du composé objet de l'exemple 61 ont été hydrogénés dans les conditions décrites dans l'exemple 29 pour donner, après recristallisation dans l'isopropanol environ 2 g du composé attendu, PF : 232 °C.

### Exemple 64

### 5,7-dihydroxy-2-(3-hydroxy-4-méthoxy phényl)-8-propyl-4H-1-benzopyran-4-one.

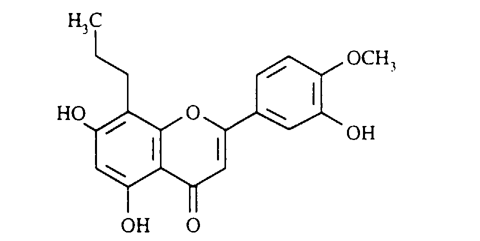

3,3 g du composé objet de l'exemple 62, dissous dans 20 ml de diméthylformamide sont hydrogénés sous une pression de 6300 hPa, en présence de 100 mg de charbon palladié à 5 % de Palladium, comme catalyseur. Après absorption de la quantité théorique d'hydrogène, le milieu est amené à sec, le résidu est repris dans l'éthanol à chaud et la solution est filtrée sur millipore. Le solvant est distillé sous pression réduite et le résidu recristallisé dans l'isopropanol pour donner 3 g du produit attendu PF : 250 °C.

### Exemple 65

### 8-allyl-5,7-dihydroxy-2-(2-allyl-3-hydroxy-4-méthoxy phényl)-4H-1-benzopyran-4-one.

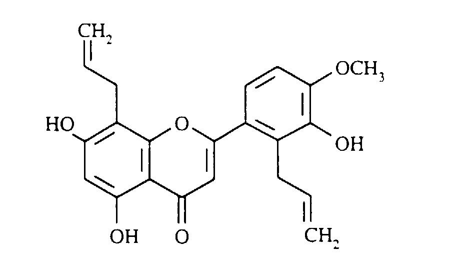

7,5 g du composé objet de l'exemple 2 sont transposés dans les conditions décrites dans l'exemple 11 étape B. Après élimination du solvant, le résidu est chromatographié sur silice en éluant avec un mélange CH₂Cl₂/CH₃COOC₂H₅ (90/10), pour donner, après réunion des fractions identiques et distillation des solvants :
- environ 2,2 g du composé attendu objet du présent exemple et
- 3,8 g du composé objet de l'exemple 66 ci-après désigné, chacun de ces composés étant utilisables tels quels.

Par recristallisation de chacun d'eux dans l'isopropanol, ont été obtenus des échantillons analytiques à l'état pur des composés objet de l'exemple 66, PF : 224 °C, et de l'exemple 65, sous forme α, PF : 120 °C (isopropanol) et sous forme β, PF : 205 °C, (CH₂Cl₂/CH₃OH).

### Exemple 66

### 6-allyl-5,7-dihydroxy-2-(2-allyl-3-hydroxy-4-méthoxy phényl)-4H-1-benzopyran-4-one.

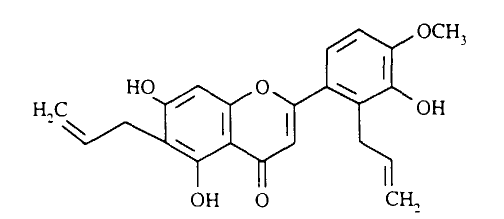

Ce composé a été préparé comme décrit dans l'exemple 65 ci-dessus, PF : 224 °C.

### Exemple 67

### 5,7-dihydroxy-2-(3-hydroxy-4-méthoxy-2-propyl phényl)-6-propyl-4H-1-benzopyran-4-one.

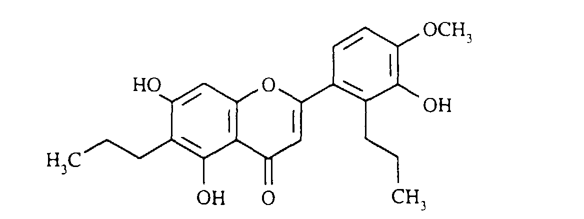

2 g du composé objet de l'exemple 66 sont hydrogénés dans les conditions décrites dans l'exemple 29 pour donner, après recristallisation dans un mélange éther/éther de pétrole, 1,8 g du composé attendu, PF : 246 °C.

### Exemple 68

### 5,7-dihydroxy-2-(3-hydroxy-4-méthoxy-2-propyl phényl)-8-propyl-4H-1-benzopyran-4-one.

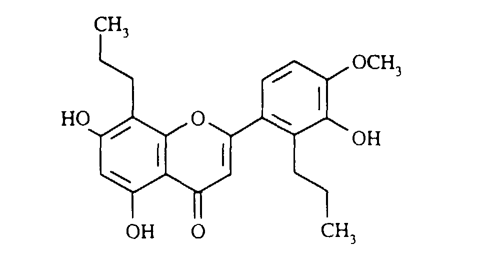

3,7 g du composé objet de l'exemple 65 ont été hydrogénés dans les conditions décrites dans l'exemple 29, pour donner 3 g du composé attendu, PF : 134 °C.

### Exemple 69

### 6,8-diallyl-5,7-dihydroxy-2-(2-allyl-3-hydroxy-4-méthoxy phényl)-4H-1-benzopyran-4-one.

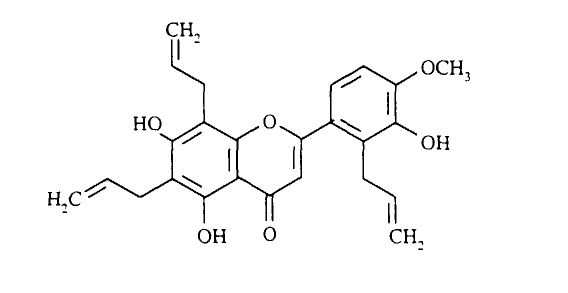

4,2 g du composé objet de l'exemple 3 sont transposés dans les conditions décrites dans l'exemple 11 étape B. Le résidu est dissout dans CH₂Cl₂ et la solution est filtrée sur 10 g de silice en éluant avec un mélange CH₂Cl₂/CH₃COOC₂H₅ (90/10).

Après récupération du filtrat, les solvants sont éliminés par distillation et le résidu est recristallisé dans un mélange éthanol/éther pour obtenir finalement 3,8 g du composé attendu, PF : 122-123 °C.

### Exemple 70

### 5,7-dihydroxy-2-(3-hydroxy-4-méthoxy-2-propyl phényl)-6,8-dipropyl-4H-1-benzopyran-4-one.

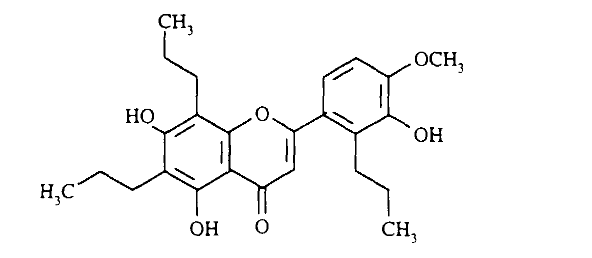

3,5 g du composé objet de l'exemple 69 sont hydrogénés dans les conditions décrites dans l'exemple 29, en utilisant l'éthanol comme solvant. Après filtration sur millipore, élimination du solvant et cristallisation du produit dans un mélange éther/éther de pétrole, il a été obtenu 3,3 g de composé attendu, PF : 170 °C.

### Exemple 71

### 5,7-dihydroxy-2-(3-allyloxy-4-méthoxy phényl)-4H-1-benzopyran-4-one

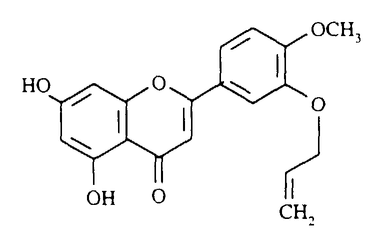

20 g de diosmétine sont dissouts dans un mélange de 400 ml de méthanol anhydre et 13,3 g de potasse en pastilles. Le mélange réactionnel est maintenu à température ambiante puis additionné goutte à goutte de 16 g de bromure d'allyle en solution dans 40 ml de méthanol sous agitation. L'agitation est maintenue ensuite pendant 48 heures, puis les solvants et produits volatils sont distillés sous pression réduite, à température ambiante. Le résidu est répris dans 400 ml de CH₂Cl₂, l'insoluble est recueilli par filtration puis recristallisé dans 440 ml d'isopropanol pour donner 19 g du composé attendu, PF : 219 °C.

### Exemple 72

### 5,7-dihydroxy-2-(2-allyl-3-hydroxy-4-méthoxy phényl)-4H-1-benzopyran-4-one

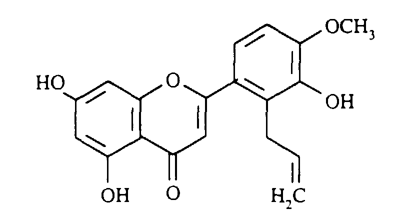

9 g du composé objet de l'exemple 71 sont transposés dans les conditions décrites dans l'exemple 11 étape B, pour obtenir, après recristallisation dans l'isopropanol, 7 g du composé attendu, PF : 234 °C.

### Exemple 73

### 7-allyloxy-5-hydroxy-2-(2-allyl-3-hydroxy-4-méthoxy phényl)-4H-1-benzopyran-4-one

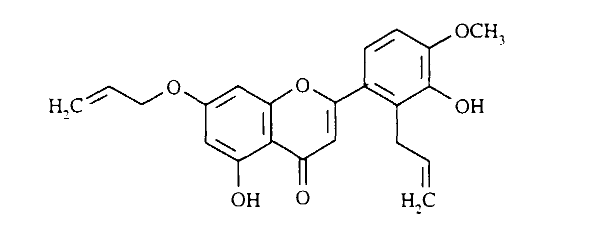

3,5 g du composé objet de l'exemple 72 sont alkylés avec du bromure d'allyle dans les conditions objet de l'exemple 1, pour donner, après recristallisation dans un mélange isopropanol/éther, 3,5 g du composé attendu, PF : 155 °C.

### Exemple 74

### 5-hydroxy-2-(3-hydroxy-4-méthoxy-2-propyl phényl)-7-propyloxy-4H-1-benzopyran-4-one

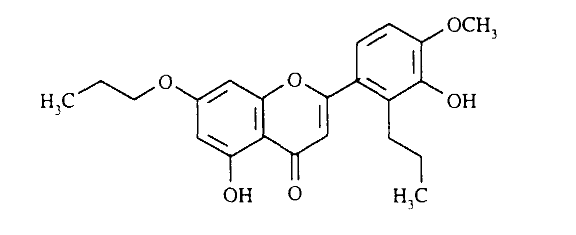

3,5 g du composé objet de l'exemple 73 sont réduits catalytiquement dans les conditions décrites dans l'exemple 43, pour donner, après recristallisaton dans un mélange isopropanol/éther, 2,8 g du composé attendu, PF : 148-149 °C.

### Exemple 75

### 5-hydroxy-7-[(2,2-diméthyl-1,3-dioxol-4-yl) méthoxy]-2-(3-allyloxy-4-méthoxy phényl)-4H-1-benzopyran-4-one

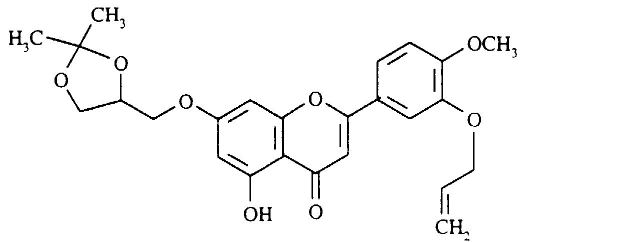

9 g du composé objet de l'exemple 71 sont alkylés avec le (R,S) p-toluènesulfonate de (2,2-diméthyl-1,3-dioxol-4 yl) méthyle, dans les conditions décrites dans l'exemple 4 pour obtenir finalement 9,1 g du composé attendu, PF : 139 °C lequel composé a fait également l'objet de l'exemple 32.

### Exemple 76

### 5,7-dihydroxy-2-(3-hydroxy-4-méthoxy-2-propyl phényl)-4H-1-benzopyran-4-one

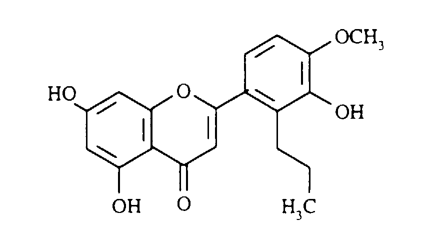

3,5 g du composé objet de l'exemple 72 sont hydrogénés dans les conditions décrites dans l'exemple 43 pour donner, après recristallisation dans l'isopropanol, 3,1 g du composé attendu, PF : 200 °C.
Ce composé est identique à celui préparé selon l'exemple 11.

### Exemple 77

### 6-allyl-5,7-diallyloxy-2-(3-allyloxy-4-méthoxy phényl)-4H-1-benzopyran-4-one.

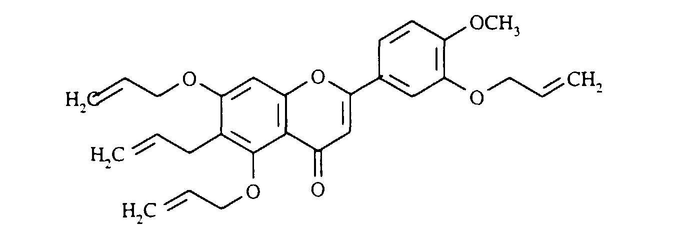

12 g de diosmétine dans 100 ml de diméthyl formamide et 15 ml de HMPA sont ajoutés très lentement à une suspension de 4,8 g d'hydrure de sodium dans 30 ml de diméthyl formamide. Le mélange est agité ensuite à 40 °C jusqu'à fin du dégagemet gazeux. On ajoute alors goutte à goutte 17,25 g de bromure d'allyle et porte à 60 °C pendant 16 heures. Après quoi, les solvants sont éliminés par distillation sous pression réduite. Le résidu, repris dans le dichlorométhane, est ensuite chromatographié sur silice en éluant avec un mélange dichlorométhane 98 % méthanol 2 %. On récupère ainsi une fraction de 7,6 g de composé très flurorescent sous 366 nm. Un échantillon analytique du composé titre est préparé par recristallisation dans l'isopropanol, PF : 107 C°.

### Exemple 78

### 7-allyloxy 6,8-diallyl-2-(2-allyl-3-hydroxy-4-méthoxyphényl)-4H-1-benzopyran-4-one.

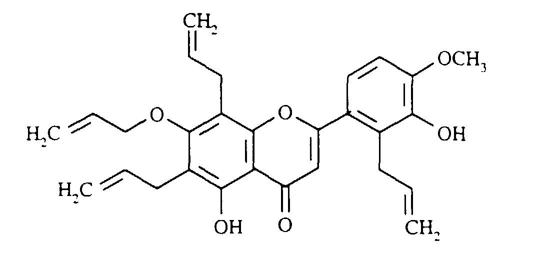

20 g du composé objet de l'exemple 77 sont transposés dans les conditions de l'exemple 11 étape B en maintenant le chauffage pendant 1 heure. Après double recristallisation dans un mélange isopropanol/diéthyléther, on récupère 9,8 g du composé attendu, PF : 125 °C.

### Exemple 79

### 5,7-bis[(2,2-diméthyl dioxol-4-yl)méthoxy]-2-(3-allyloxy-4-méthoxyphényl)-4H-1-benzopyran-4-one.

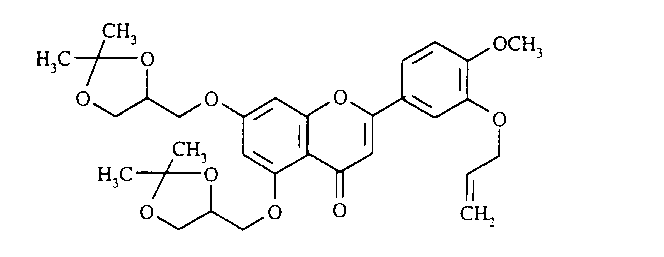

4 g du composé objet de l'exemple 32 sont alcoylés avec du p-toluène sulfonate de 2,2-diméthyl-1,3-dioxol-4-yl)méthyle dans les conditions de l'exemple 19 étape B. On obtient ainsi 3,6 g du composé attendu, PF : 123 °C.

### Exemple 80

### 5,7-bis[(2,2-diméthyl dioxol-4-yl)méthoxy]-2-(2-allyl-3-hydroxy-4-méthoxy phényl) 4H-1-benzopyran-4-one.

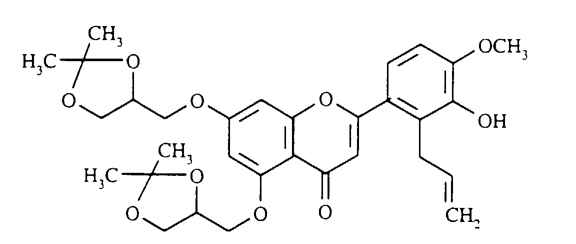

le composé objet de l'exemple 79 est transposé dans les conditions de l'exemple 11 étape B. On obtient ainsi le composé titre utilisable tel quel.
Un échantillon analytique de ce composé est préparé par recristallisation dans l'isopropanol, PF : 98-100 °C.

### Exemple 81

### 5,7-bis[(2,2-diméthyl dioxol-4-yl)méthoxy]-2-(3-hydroxy-4-méthoxy-2-n-propylphényl) 4H-1-benzopyran-4-one.

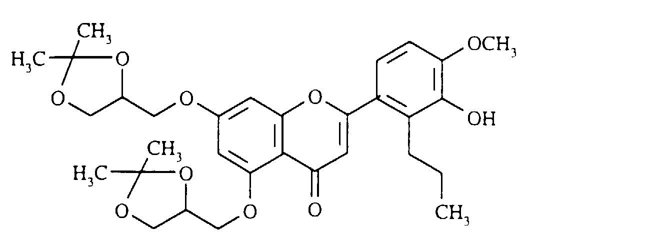

Le composé objet de l'exemple 80 est hydrogéné dans les conditions de l'exemple 43 pour donner le composé attendu utilisable tel quel.

### Exemple 82

### 5,7-bis(2,3-dihydroxypropyloxy)-2-(3-hydroxy-4-méthoxy-2-propylphényl)-4H-1-benzopyran-4-one.

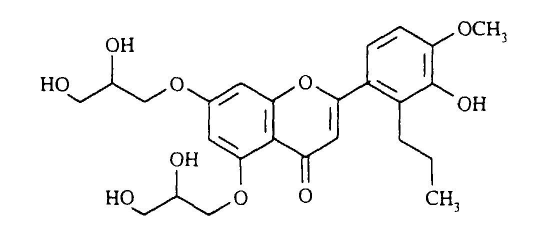

le composé objet de l'exemple 81 est hydrolysé dans les conditions de l'exemple 16. Le produit titre obtenu est recristallisé dans le mélange isopropanol, éther, éther de pétrole, PF : 183-188 °C.

### Exemple 83

### 5-méthoxy-7-[(2,2-diméthyl dioxol-4-yl)-méthoxy]-2-(3,4-diméthoxy-2-propylphényl)-4H-1-benzopyran-4-one.

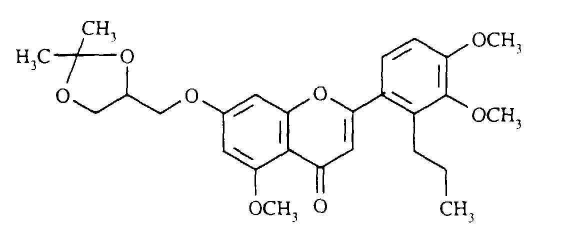

Le composé objet de l'exemple 43 est alcoylé dans les conditions de l'exemple 14, en utilisant de l'hydrure de sodium. Le composé titre obtenu est purifié par recristallisation dans le mélange cyclohexane, acétate d'éthyle, PF : 205 °C (déc).

### Exemple 84

### 5-méthoxy 7-(2,3-dihydroxy propyloxy) 2-(3,4-diméthoxy-2-n.propylphényl)-4H-1-benzopyran-4-one.

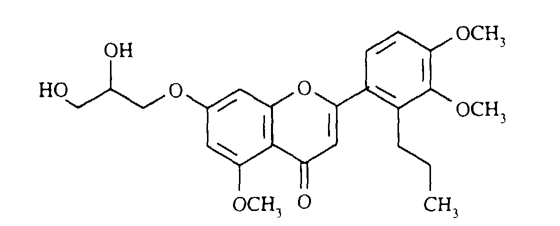

Le composé objet de l'exemple 83 est hydrolysé dans les conditions de l'exemple 10 pour donner le composé titre, PF : 221 °C.

### Exemple 85

### 5,7-bis propargyloxy-2-(4-méthoxy-3-propargyloxyphényl)-4H-1-benzopyran-4-one.

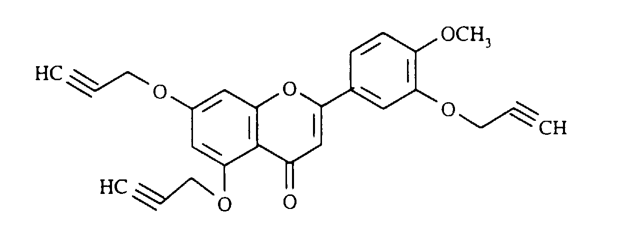

30 g de diosmétine sont alcoylés avec du bromure de propargyle dans les conditions de l'exemple 3. On obtient ainsi après recristallisation dans l'isopropanol 32 g du composé titre à l'état pur, PF : 196 °C.

### Exemple 86

### 5,7-dihydroxy-6,8-bis (1,2-dideutéroallyl) 2-[(4-méthoxy 3-hydroxy 2-(1,2-dideutéroallyl) phényl] 4H-1-benzopyran-4-one.

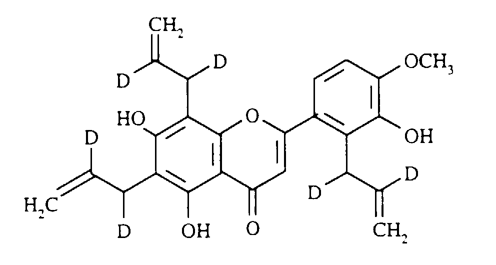

### Etape A - Deutération

4 g du composé de l'exemple 85 sont dissouts dans 60 ml de diméthylformamide, puis réduits par du deutérium gazeux à pression atmosphérique en présence de 0,7 g de catalyseur de Lindlar. Après absorption de 810 ml (125 %) de deutérium, le solvant est éliminé par distillation sous pression réduite ; le résidu huileux est filtré sur 80 g de silice en éluant avec un mélange dichlorométhane 99,5 % méthanol 0,5 %. On obtient ainsi 3,4 g de composé hexadeutéré (pureté > 90 %), utilisé tel quel dans l'étape ultérieure.

### Etape B - Transposition

Le composé obtenu dans l'étape A est porté à reflux sous azote dans 34 ml de trichlorobenzène. Le solvant est ensuite distillé sous pression réduite et le résidu est chromatographié sur 90 g de silice en éluant avec un mélange toluène 95 %, acétate d'éthyle 5 %. Le produit obtenu après réunion des fractions pures est ensuite recristallisé dans l'isopropanol pour donner 2 g de produit titre à l'état pur, PF : 128 °C.

### Exemple 87 : ETUDE PHARMACOLOGIQUE

### 1/ Activité anti-hyperperméabilité

Elle a été déterminée par l'effet des composés de l'invention sur l'extravasation de FITC-dextran dans la microcirculation du hamster cheek pouch.
Le hamster cheek pouch est un modèle expérimental permettant d'étudier de façon quantitative la perméabilité de macromolécules en utilisant du dextran marqué à la fluorescéine (FITC-dextran).

Les expériences ont été effectuées sur des hamsters mâles dont le poids est de 85 à 120 g. Les animaux sont anesthésiés avec du pentobarbital sodique (60 mg/kg i.p.). Pendant l'expérience, l'anesthésie est maintenue par de l'α-chloralose (100 mg/kg) introduite par un cathéter dans l'artère fémorale. Les animaux respirent spontanément par une canule trachéale et la température corporelle est maintenue à 37,5 °C à l'aide d'un "heating pad" contrôlé par un thermistor rectal.

Après l'anesthésie initiale, le cheek pouch est préparé selon la méthode de Duling (Microvasc. Res,.5, 423-429, 1973) et Svensjö et al. (Uppsala. J. Med. Sci., 83, 71-79, 1978). La préparation est montée dans une cuve expérimentale et superfusée à 6 ml/min par une solution physiologique contenant du Hepes. La température de la solution physiologique est maintenue à 36,5 °C et un courant de gaz (5 % CO₂ - 95 % N₂) est appliqué au-dessus de la cuve expérimentale afin de maintenir le niveau de la pO₂ de la solution entre 12 et 15 mmHg et le pH à 7,4.

Trente minutes après le montage de la préparation, le FITC-dextran (150, 000 dalton, 50 mg/ml) est administré par voie intraveineuse chez l'animal à la dose de 250 mg/kg. La préparation est observée par la méthode de "intravital microscopy" en lumière UV à l'aide d'un microscope Ortholux II de Leitz.

Après l'injection de FITC-dextran, la préparation est soumise soit à une application locale d'un agent induisant la perméabilité : l'histamine (2x10⁻⁶ M) ou le leucotriène B₄ (LTB₄ ; 10⁻⁸ M), soit à une période d'ischémie (30 mn)-reperfusion.

L'application de ces agents et de la période d'ischémie provoque des "fuites" (visibles comme des petites plaques fluorescentes) dans toute la préparation. Le nombre de fuites est déterminé à plusieurs intervalles après l'administration de l'histamine ou du LTB₄ (de 2 à 30 mn) et à 30 mn de reperfusion après l'ischémie et exprimé en nombre de fuites par cm². Les préparations sont exposées à plusieurs reprises (séparées d'au moins 30 minutes) à une application locale d'histamine ou de LTB₄.
Deux types d'études ont été effectués afin de déterminer l'activité per os ou l'activité après administration i.v. des composés de la présente invention.

### 1.1 Activité per os

Les animaux sont traités par gavage, trente minutes avant l'anesthésie, avec différentes doses (de 1 à 100 mg/kg) d'un des composés de l'invention mis en suspension dans la gomme arabique. La dose du produit est contenue dans 0,2 ml. Les animaux contrôles reçoivent un gavage avec 0,2 ml de gomme arabique (placébo).

### 1.1.1 Activité per os à 100 mg/kg

Les composés de l'invention administrés à 100 mg/kg p.o. chez le hamster, diminuent de façon significative (t-test ; ∗p<0,05) et durable, l'extravasation de macromolécules provoquée par l'histamine ou le LTB₄ dans la microcirculation. Les résultats obtenus avec l'histamine sont récapitulés dans le tableau 1. Ces résultats sont supérieurs à ceux obtenus dans les mêmes conditions opératoires, avec la substance de référence, la Proxérutine.

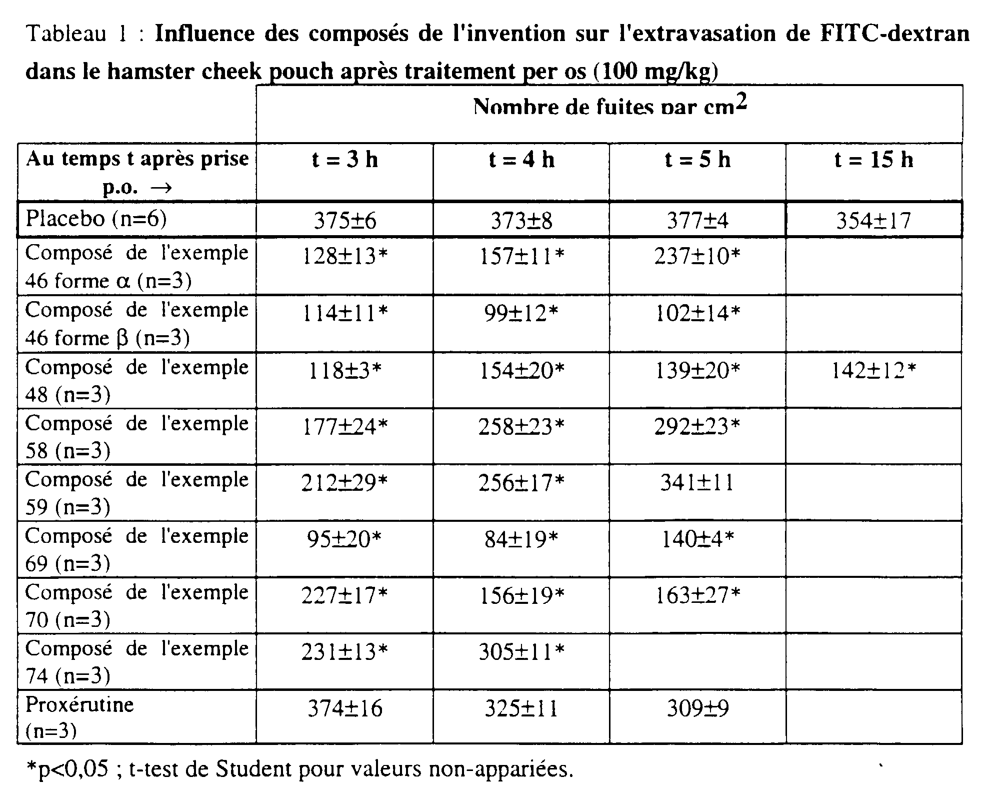

### 1.1.2 Activité per os : effet dose

Les composés de l'invention, administré à des doses de 1 à 100 mg/kg p.o. chez le hamster, diminuent de façon significative et dose-dépendante, à partir de la plus faible dose de 1 mg/kg, l'extravasation de macromolécules provoquée par l'histamine dans la microcirculation. Les résultats obtenus 3 heures après l'administration p.o sont récapitulés dans le tableau 2.

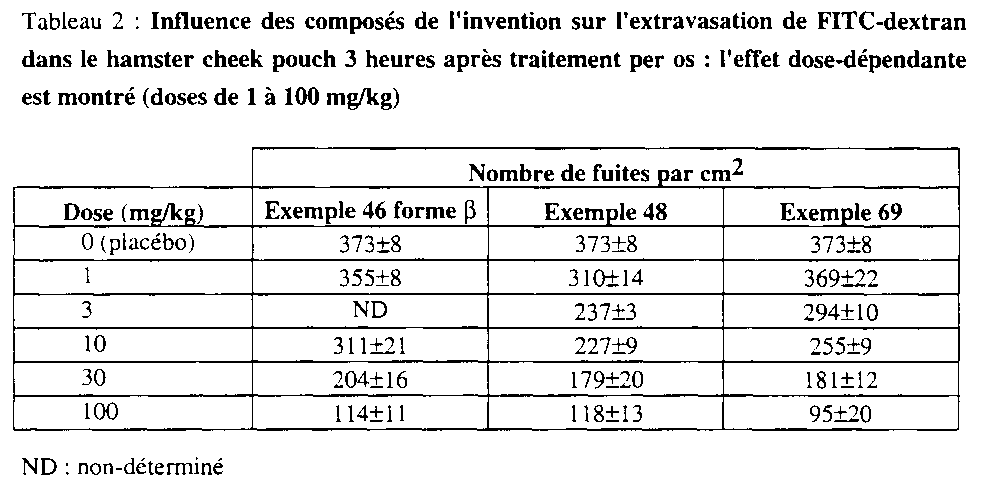

### 1.1.3 Activité per os : fuites provoquées par l'ischémie/reperfusion

Les composés de l'invention, administrés à des doses de 1 à 30 mg/kg p.o. chez le hamster, diminuent de façon significative et dose-dépendante, dès la plus faible dose de 1 mg/kg, l'extravasation de macromolécules provoquée par une période d'ischémie de 30 mn. Les résultats obtenues 3 heures après l'administration p.o. des composés sont réunis dans le tableau 3.

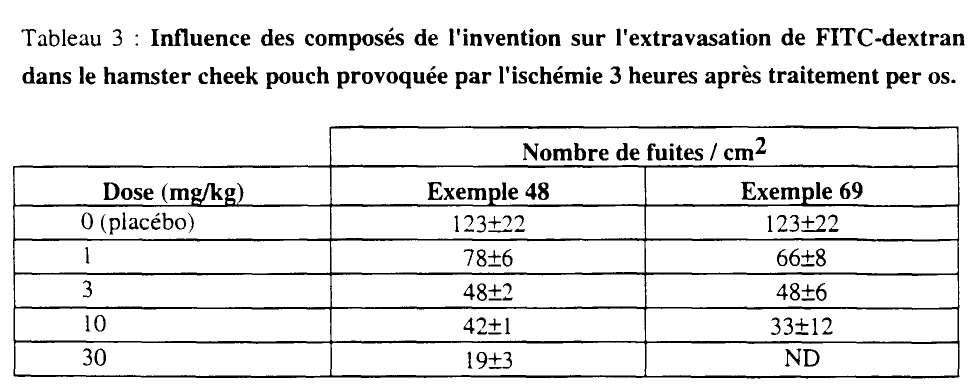

### 1.2 Activité i.v.

Les composés de l'invention dissous dans la gomme arabique sont admininistrés à 10 mg/kg i.v. (0,2 ml de la solution + 0,2 ml de la solution de NaCl 0,9 %). Ils diminuent de façon significative et durable,l'extravasation de macromolécules provoquée par l'histamine dans la microcirculation. Les résultats sont indiqués dans le tableau 4.

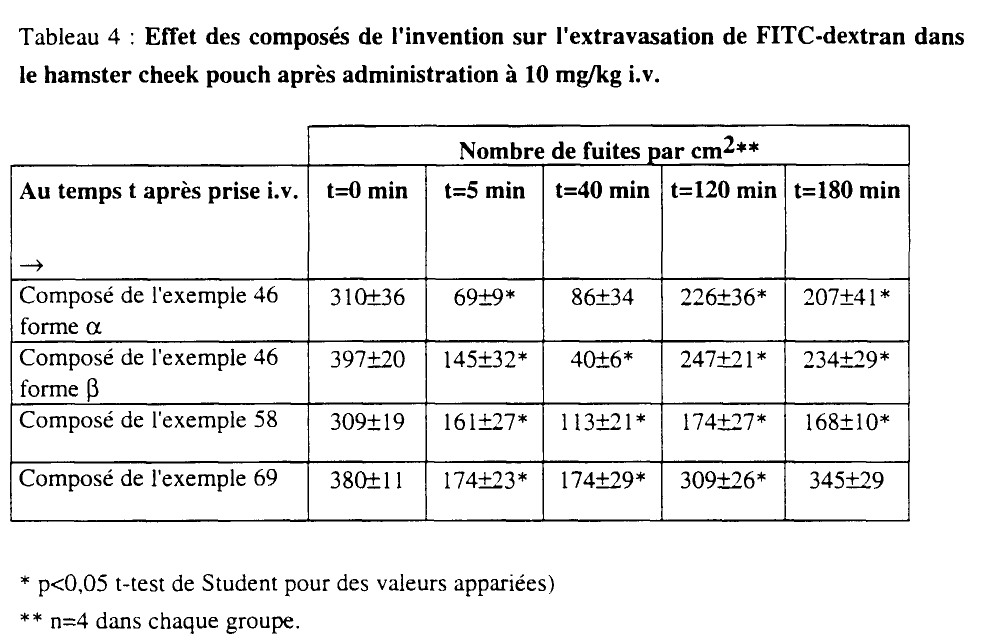

### 2/ Activité anti-inflammatoire

### 2.1 Activité anti-inflammatoire sur un modèle d'inflammation trachéo-bronchique induit par inhalation de LPS.

Le test consiste en l'étude de l'augmentation de la cellularité au niveau du liquide de lavage alvéolaire de cobaye (Cobayes Hartley, n=8 par lot, un lot témoin et un lot traité) après exposition pendant vingt minutes à une solution de LPS (E. COLI) à une concentration de 1,25µg/ml. Les animaux sont traités 24 heures avant l'exposition à l'aérosol et le jour même de l'exposition. Les animaux sont sacrifiés 24 heures après l'exposition et un lavage broncho-alvéolaire est effectué. Le critère de jugement est la quantification du nombre total de cellules et du nombre de polymorphonucléaires dans le liquide de lavage.

Ainsi, le produit objet de l'exemple 16 à la dose 100 mg/kg par jour PO a entraîné une diminution significative de la cellularité de 25 % et du nombre de polymorphonucléaires de 37 %.

### 2.2 Activité anti-inflammatoire sur le modèle d'oedème d'oreille de souris induit par application topique d'Acide Arachidonique.

Le test consiste en l'étude chez la souris (Souris CD1, mâles (n=6/lot) de l'augmentation du poids de l'oreille induit par application topique d'acide arachidonique (1 mg, délai 30 minutes) comparativement au poids de l'oreille témoin controlatérale.
Le critère de jugement est la différence de poids entre oreille inflammée et oreille témoin. Les résultats sont exprimés en % d'inhibition par rapport à un lot témoin n'ayant pas reçu de produit.
Ainsi, le produit, objet de l'exemple 16, à la dose de 20 mg/kg (PO 1 heure avant application de l'acide arachidonique) a entrainé une inhibition significative de l'oedème de l'oreille de souris de 25 %.

## Revendications

1. Les dérivés de la diosmétine de formule I : 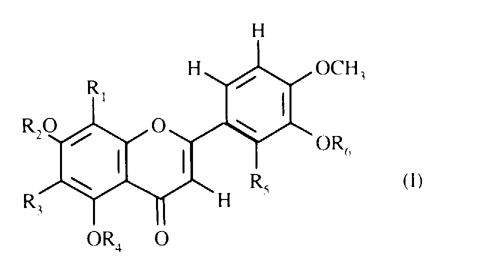 dans laquelle :
- R₁ représente un atome d'hydrogène ou un radical propyle, allyle ou 1,2-dideutéroallyle;
- R₂ représente un atome d'hydrogène ou un radical propyle, allyle. propargyle, 2,3-dihydroxypropyle, (2,2-diméthyl-1,3-dioxol-4-yl) méthyle ou 3-acétyloxy-2-hydroxypropyle ;
- R₃ représente un atome d'hydrogène ou un radical propyle, allyle ou 1,2-dideutéroallyle ;
- R₄ représente un atome d'hydrogène ou un radical méthyle, propyle, allyle, propargyle, 2,3-dihydroxypropyle, (2,2-diméthyl-1,3-dioxol-4-yl) méthyle ou un radical de formule -COR'₄ [dans laquelle R'₄ représente un radical alkyle de 1 à 5 atomes de carbone en chaîne droite ou ramifiée ou un radical phényle] ;
- R₅ représente un atome d'hydrogène ou un radical propyle, allyle ou 1,2-dideutéroallyle, et
- R₆ représente un atome d'hydrogène ou un radical méthyle. propyle, allyle, propargyle, 2,3-dihydroxypropyle, (2,2-diméthyl-1,3-dioxol-4-yl) méthyle, in radical de formule -COR'₆ [dans laquelle R'₆ représente un radical alkyle de 1 à 5 atomes de carbone en chaîne droite ou ramifiée ou un radical phénylel ou un radical de formule A : 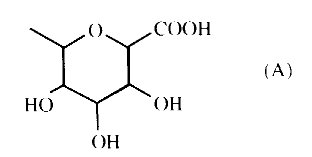
à condition que :
- l'un au moins des R₁, R₂, R₃, R₄, R₅ et R₆ prenne une signification autre qu'hydrogène, et que
- si R₁, R₂ et R₃ représentent chacun simultanément un atome d'hydrogène, alors R₄ représente aussi un atome d'hydrogène ;
- si R₁, R₂, R₃, R₄ et R₅ représentent chacun simultanément un atome d'hydrogène, alors R₆ ne représente pas le radical de formule A ;
ainsi que leurs diastéréoisomères et/ou énantiomères.

2. Un composé de la revendication 1 qui est le 7-allyloxy-5-hydroxy-2-(3-allyloxy-4-méthoxy phényl)-4H-1-benzopyran-4-one.

3. Un composé de la revendication 1 qui est le 5,7-dihydroxy-2-{3-[(2,3-dihydroxy propyl oxy)4-méthoxy phényl}-4H-1-benzopyran4-one.

4. Un composé de la revendication 1 qui est le (R,S) 5-hydroxy-2-(3-hydroxy4-méthoxy phényl)-7-(2,3-dihydroxy propyl oxy)-4H-1-benzopyran-4-one.

5. Un composé de la revendication 1 qui est le 5,7-di-(2,3-dihydroxy propyl oxy)-2-(3-hydroxy4-méthoxy phényl)-4H-1-benzopyran-4-one.

6. Un composé de la revendication 1 qui est le 5-hydroxy-2-(4-méthoxy-3-pivaloyloxy phényl)-7-(2,3-dihydroxy propyloxy)4H-1-benzopyran-4-one.

7. Un composé de la revendication 1 qui est le 5-allyloxy-2-(3-allyloxy-4-méthoxy phényl)-7-(2,3-dihydroxy propyloxy)-4H-1-benzopyran-4-one.

8. Un composé de la revendication 1 qui est le 6-allyl-5-hydroxy-2-(2-allyl-3-hydroxy-4-méthoxy phényl)-7-(2,3-dihydroxy propyloxy)4H-1-benzopyran4-one.

9. Un composé de la revendication 1 qui est le 5-hydroxy-2-(3-hydroxy-4-méthoxy-2-propyl phényl)-6-propyl-7-(2,3-dihydroxy propyloxy)-4H-1-benzopyran-4-one.

10. Un composé de la revendication 1 qui est le (R,S) 5-hydroxy-2-(3-hydroxy-4-méthoxy-2-propyl phényl)-7-(2,3-dihydroxy propyl oxy)-4H-1-benzopyran-4-one, sous ses deux formes α et β.

11. Un composé de la revendication 1 qui est le (R) 5-hydroxy-2-(3-hydroxy-4-méthoxy-2-propyl phényl)-7-(2,3-dihydroxy propyl oxy)-4H-1-benzopyran-4-one.

12. Un composé de la revendication 1 qui est le (S) 5-hydroxy-2-(3-hydroxy-4-méthoxy-2-propyl phényl)-7-(2,3-dihydroxy propyloxy)-4H-1-benzopyran4-one.

13. Un composé de la revendication 1 qui est le (R,S) 5-hydroxy-2-(3-hydroxy-4-méthoxy-2-propyl phényl)-7-(3-acétyloxy-2-hydroxy propyloxy)4H-1-benzopyran-4-one.

14. Un composé de la revendication 1 qui est le 5-hydroxy-2-[4-méthoxy-2-propyl-3-(6-carboxy-3,4,5-trihydroxy tétrahydropyran-2-yl oxy) phényl]-7-(2,3-dihydroxy propyloxy)4H-1-benzopyran-4-one.

15. Un composé de la revendication 1 qui est le 5-hydroxy-2-[4-méthoxy-3-(2,3-dihydroxy propyloxy) phényl]-7-(2,3-dihydroxy propyloxy)4H-1-benzopyran-4-one.

16. Un composé de la revendication 1 qui est le 6,8-diallyl-5,7-dihydroxy-2-(2-allyl-3-hydroxy-4-méthoxy phényl)4H-1-benzopyran-4-one.

17. Un composé de la revendication 1 qui est le 5,7-dihydroxy-2-(3-hydroxy-4-méthoxy-2-propyl phényl)-6,8-dipropyl-4H-1-benzopyran-4-one.

18. Le procédé de préparation des composés de la revendication 1 caractérisé en ce que la diosmétine de formule II : 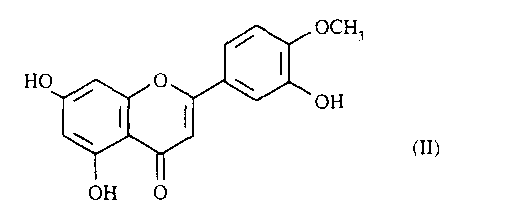 est traitée :
A/ soit par un composé de formule III : 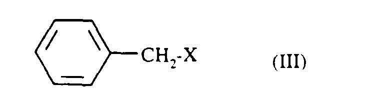 dans laquelle X représente un atome d'halogène ou un radical tosyloxy,
pour obtenir un composé de formule IV : 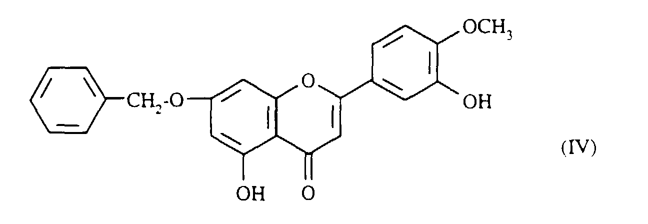 lequel est traité :
a) soit par un halogénure d'allyle de formule H₂C=CH-CH₂-Hal dans laquelle Hal représente un atome de chlore ou de brome,
pour donner un composé de formule V : 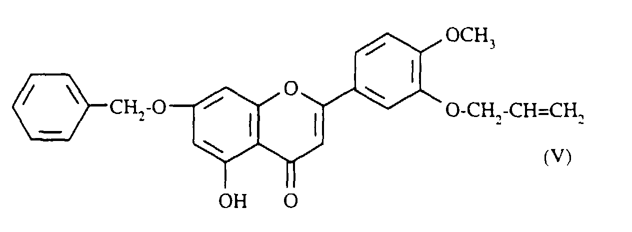 lequel par transposition donne le composé de formule VI : 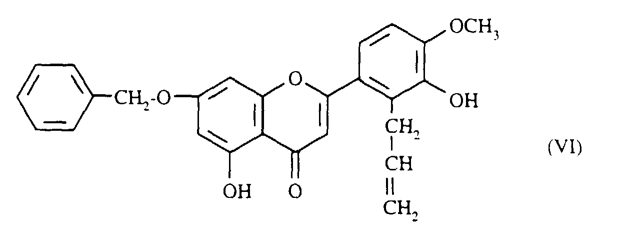 qui, par hydrogénation, donne le composé de formule Ia : 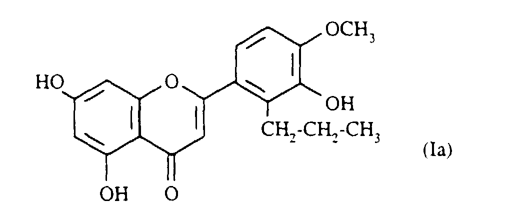 qui, traité à son tour par un halogénure d'allyle de formule H₂C=CH-CH₂-Hal dans laquelle Hal représente un atome de chlore ou de brome, conduit au composé de formule (Ib) : 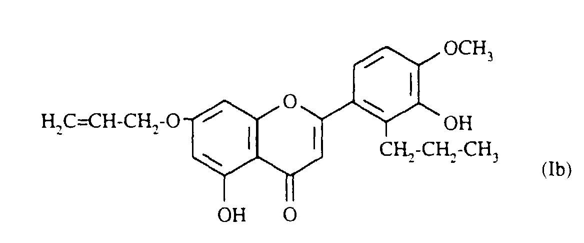 lequel par hydrogénation donne la composé de formule Ic : 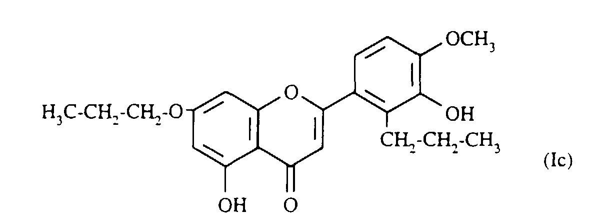
b) soit par le tosylate de (2,2-diméthyl-1,3-dioxol-4-yl) méthyle de formule : 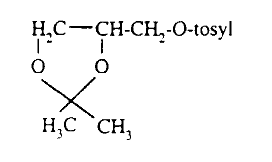 pour donner le composé de formule VII : 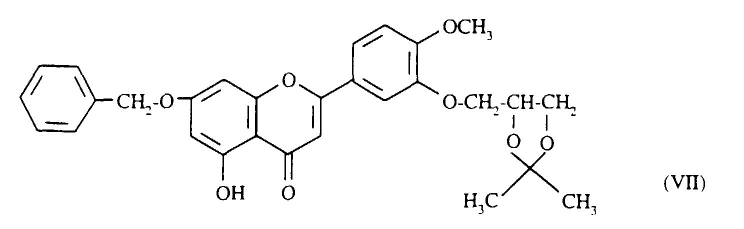 lequel par hydrogénation donne le composé de formule Id : 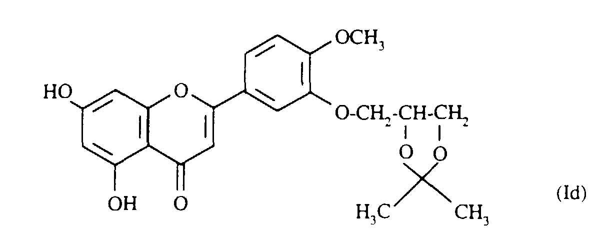 qui, traité par acide acétique et H₂O donne le composé de formule Ie : 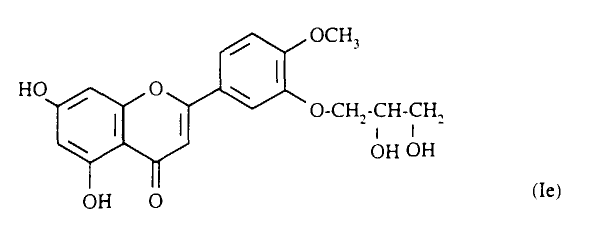 ou
B/ soit par un composé de formule III' :
R'₂-X (III')
dans laquelle :
- R'₂ représente un radical allyle, propargyle ou (2,2-diméthyl-1,3-dioxol-4-yl) méthyle, et
- X a la signification précédemment définie pour obtenir, selon la quantité du composé III' par rapport à celle du composé II, l'un ou l'autre des composés de formule If, Ig et Ih : 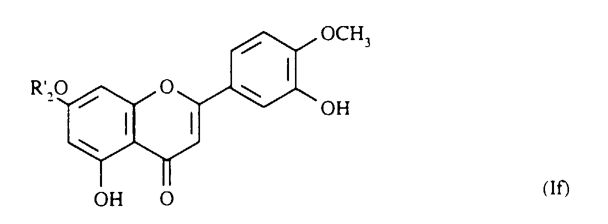 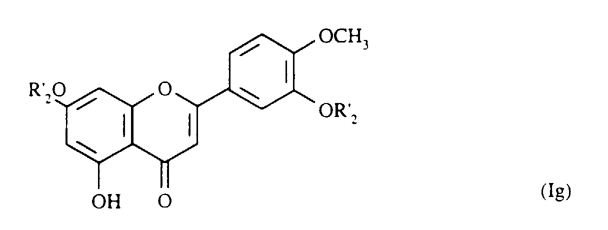 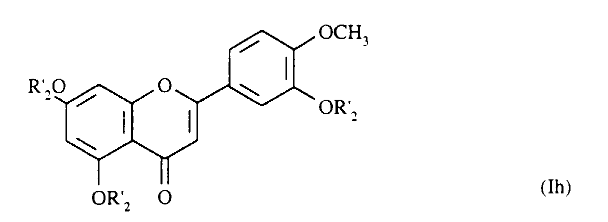
lesquels composés peuvent être soumis à des réactions ultérieures choisies parmi les réactions d'alkylation, transposition, estérification, réduction et hydrolyse pour donner les produits de formule I autre que ceux de formule la à Ih dont la préparation est précédemment décrite.

19. Les compositions pharmaceutiques contenant comme principe actif un dérivé selon l'une quelconque des revendications 1 à 17, mélangé ou associé à un ou plusieurs excipients pharmaceutiques appropriés.

20. Les compositions pharmaceutiques selon la revendication 19 présentées sous une forme convenant dans le traitement de l'insuffisance veineuse chronique.

## Patentansprüche

1. Diosmetin-Derivate der Formel I: 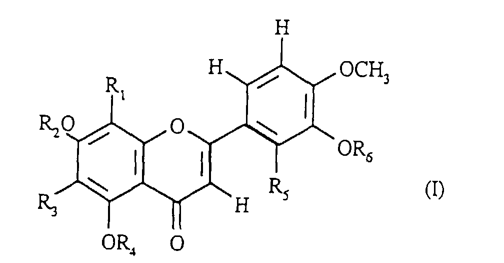 in der:
- R₁ ein Wasserstoffatom oder eine Propyl-, Allyl- oder 1,2-Dideuteroallylgruppe;
- R₂ ein Wasserstoffatom oder eine Propyl-, Allyl-, Propargyl-, 2,3-Dihydroxypropyl-, (2,2-Dimethyl-1,3-dioxol-4-yl)-methyl- oder 3-Acetyloxy-2-hydroxypropylgruppe;
- R₃ ein Wasserstoffatom oder eine Propyl-, Allyl- oder 1,2-Dideuteroallylgruppe;
- R₄ ein Wasserstoffatom oder eine Methyl-, Propyl-. Allyl-, Propargyl-, 2,3-Dihydroxypropyl-, (2,2-Dimethyl-1,3-dioxol-4-yl)-methylgruppe oder eine Gruppe der Formel -COR'₄ [in der R'₄ eine Alkylgruppe mit 1 bis 5 Kohlenstoffatomen in gerader oder verzweigter Kette oder eine Phenylgruppe darstellt];
- R₅ ein Wasserstoffatom oder eine Propyl-, Allyl- oder 1,2-Dideuteroallylgruppe, und
- R₆ ein Wasserstoffatom oder eine Methyl-, Propyl-, Allyl-, Propargyl-, 2,3-Dihydroxypropyl-, (2,2-Dimethyl-1,3-dioxol-4-yl)-methylgruppe, eine Gruppe der Formel -COR'₆ [in der R'₆ eine Alkylgruppe mit 1 bis 5 Kohlenstoffatomen in gerader oder verzweigter Kette oder eine Phenylgruppe darstellt] oder eine Gruppe der Formel A: 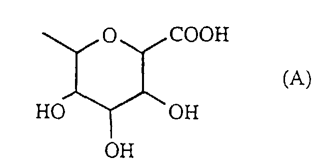 mit der Maßgabe bedeuten, daß:
- mindestens eine der Gruppen R₁, R₂, R₃, R₄, R₅ und R₆ eine von Wasserstoff verschiedene Bedeutung besitzt und
- wenn R₁, R₂ und R₃ jeweils gleichzeitig ein Wasserstoffatom bedeuten, R₄ ebenfalls ein Wasserstoffatom darstellt;
- wenn R₁, R₂, R₃, R₄ und R₅ jeweils gleichzeitig ein Wasserstoffatom darstellen, R₆ nicht die Gruppe der Formel A bedeutet;
sowie deren Diastereoisomere und/oder Enantiomere.

2. Verbindung nach Anspruch 1, nämlich 7-Allyloxy-5-hydroxy-2-(3-allyloxy-4-methoxy-phenyl)-4H-1-benzopyran-4-on.

3. Verbindung nach Anspruch 1, nämlich 5,7-Dihydroxy-2-{3-[(2,3-dihydroxypropyloxy)-4-methoxy-phenyl}-4H-1-benzopyran-4-on.

4. Verbindung nach Anspruch 1, nämlich (R,S)-5-Hydroxy-2-(3-hydroxy-4-methoxy-phenyl)-7-(2,3-dihydroxy-propyloxy)-4H-1-benzopyran-4-on.

5. Verbindung nach Anspruch 1, nämlich 5,7-Di-(2,3-dihydroxy-propyloxy)-2-(3-hydroxy-4-methoxy-phenyl)-4H-1-benzopyran-4-on.

6. Verbindung nach Anspruch 1, nämlich 5-Hydroxy-2-(4-methoxy-3-pivaloyloxy-phenyl)-7-(2,3-dihydroxy-propyloxy)-4H-1-benzopyran-4-on.

7. Verbindung nach Anspruch 1, nämlich 5-Allyloxy-2-(3-allyloxy-4-methoxyphenyl)-7-(2,3-dlhydroxy-propyloxy)-4H-1-benzopyran-4-on.

8. Verbindung nach Anspruch 1, nämlich 6-Allyl-5-hydroxy-2-(2-allyl-3-hydroxy-4-methoxy-phenyl)-7-(2,3-dihydroxy-propyloxy)-4H-1-benzopyran-4-on.

9. Verbindung nach Anspruch 1, nämlich 5-Hydroxy-2-(3-hydroxy-4-methoxy-2-propyl-phenyl)-6-propyl-7-(2,3-dihydroxy-propyloxy)-4H-1-benzopyran-4-on.

10. Verbindung nach Anspruch 1, nämlich (R,S)-5-Hydroxy-2-(3-hydroxy-4-methoxy-2-propyl-phenyl)-7-(2,3-dihydroxy-propyloxy)-4H-1-benzopyran-4-on in der α- und der β-Form.

11. Verbindung nach Anspruch 1, nämlich (R)-5-Hydroxy-2-(3-hydroxy-4-methoxy-2-propyl-phenyl)-7-(2,3-dihydroxy-propyloxy)-4H-1-benzopyran-4-on.

12. Verbindung nach Anspruch 1, nämlich (S)-5-Hydroxy-2-(3-hydroxy-4-methoxy-2-propyl-phenyl)-7-(2,3-dihydroxy-propyloxy)-4H-1-benzopyran-4-on.

13. Verbindung nach Anspruch 1, nämlich (R,S)-5-Hydroxy-2-(3-hydroxy-4-methoxy-2-propyl-phenyl)-7-(3-acetyloxy-2-hydroxy-propyloxy)-4H-1-benzopyran-4-on.

14. Verbindung nach Anspruch 1, nämlich 5-Hydroxy-2-[4-methoxy-2-propyl-3-(6-carboxy-3,4,5-trihydroxy-tetrahydropyran-2-yl-oxy)-phenyl)-7-(2,3-dihydroxypropyloxy)-4H-1-benzopyran-4-on.

15. Verbindung nach Anspruch 1, nämlich 5-Hydroxy-2-[4-methoxy-3-(2,3-dihydroxy-propyloxy)-phenyl]-7-(2,3-dihydroxy-propyloxy)-4H-1-benzopyran-4-on.

16. Verbindung nach Anspruch 1, nämlich 6,8-Diallyl-5,7-dihydroxy-2-(2-allyl-3-hydroxy-4-methoxy-phenyl)-4H-1-benzopyran-4-on.

17. Verbindung nach Anspruch 1, nämlich 5,7-Dihydroxy-2-(3-hydroxy-4-methoxy-2-propyl-phenyl]-6,8-dipropyl-4H-1-benzopyran-4-on.

18. Verfahren zur Herstellung der Verbindungen nach Anspruch 1, dadurch gekennzeichnet, daß man Diosmetin der Formel II: 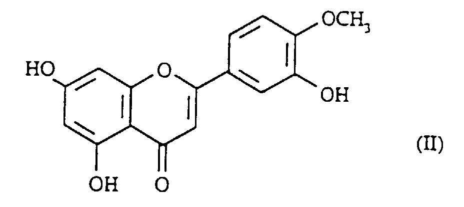
A/ entweder mit einer Verbindung der Formel III: 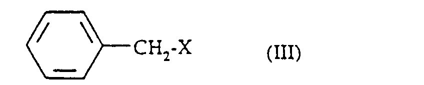 in der X ein Halogenatom oder eine Tosyloxygruppe darstellt, behandelt zur Bildung einer Verbindung der Formel IV: 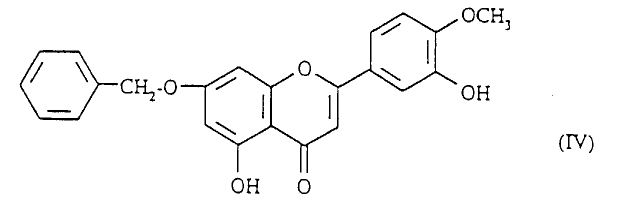 welche:
a) entweder mit einem Allylhalogenid der Formel H₂C=CH-CH₂-Hal, in der Hal ein Chlor- oder Bromatom darstellt,
behandelt wird zur Bildung einer Verbindung der Formel V: 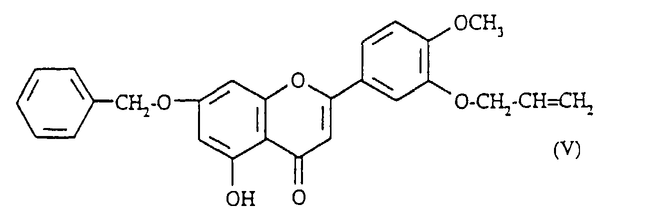 welche durch Umlagerung die Verbindung der Formel VI liefert: 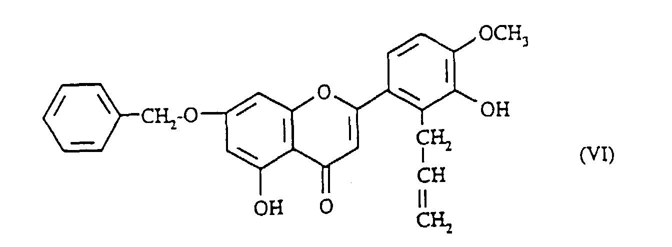 welche durch Hydrierung zu der Verbindung der Formel Ia führt: 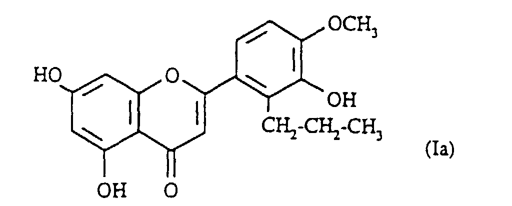 welche ihrerseits durch Behandlung mit einem Allylhalogenid der Formel H₂C=CH-CH₂-Hal, in der Hal ein Chlor- oder Bromatom darstellt, zu der Verbindung der Formel (Ib) führt: 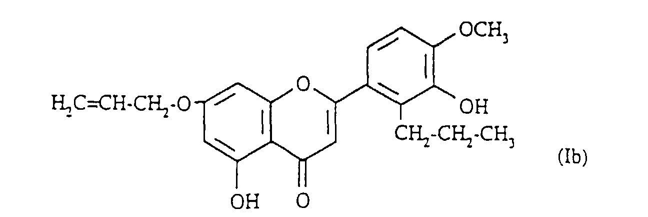 welche durch Hydrierung die Verbindung der Formel Ic ergibt: 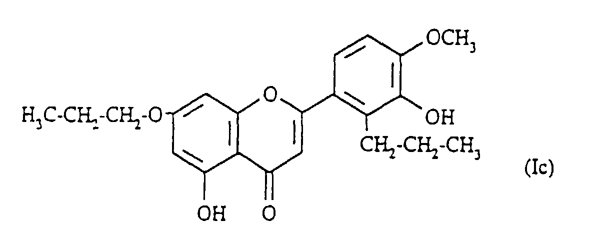
b) oder mit (2,2-Dimethyl-1,3-dioxol-4-yl)-methyl-tosylat der Formel: 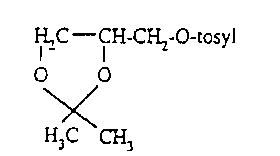 behandelt wird zur Bildung der Verbindung der Formel VII: 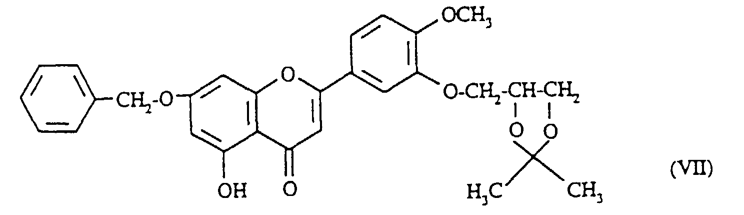 welche durch Hydrierung die Verbindung der Formel Id ergibt: 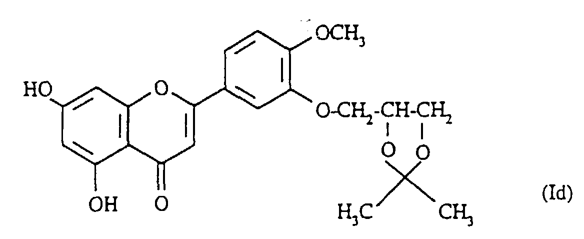 welche durch Behandlung mit Essigsäure und H₂O die Verbindung der Formel Ie liefert: 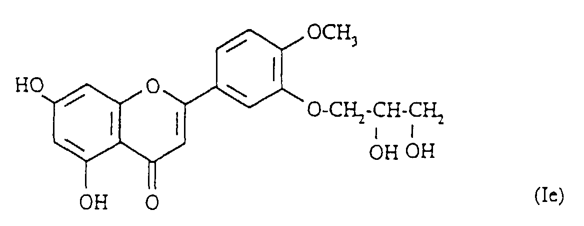
B/ oder mit einer Verbindung der Formel III' behandelt:
R'₂-X (III')
in der:
- R'₂ eine Allyl-, Propargyl- oder (2,2-Dimethyl-1,3-dioxol-4-yl)-methylgruppe darstellt und
- X die oben angegebenen Bedeutungen besitzt, so daß man in Abhängigkeit von der Menge der Verbindung III' bezogen auf die Menge der Verbindung II eine der Verbindungen der Formeln If, Ig und Ih erhält: 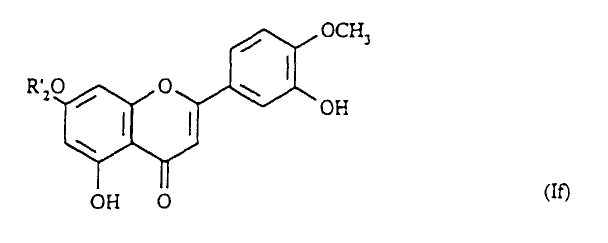 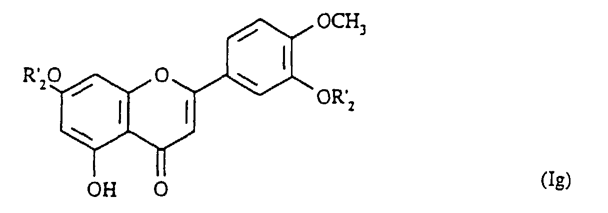 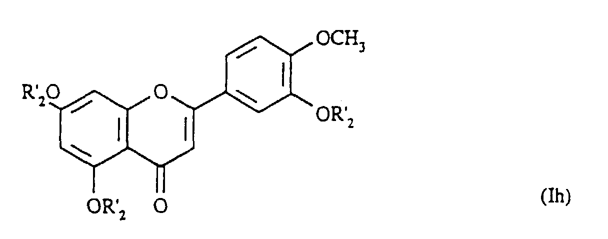
welche Verbindungen weiteren Behandlungen, ausgewählt aus Alkylierungs-, Umlagerungs-, Veresterungs-, Reduktions- und Hydrolyse-Reaktionen unterworfen werden können zur Bildung der Produkte der Formel I, die von jenen der Formel Ia bis Ih verschieden sind, deren Herstellung oben beschrieben worden ist.

19. Pharmazeutische Zubereitungen enthaltend als Wirkstoff ein Derivat nach einem der Ansprüche 1 bis 17 in Mischung oder Kombination mit einem oder mehreren pharmazeutisch geeigneten Trägermaterialien.

20. Pharmazeutische Zubereitungen nach Anspruch 19 in einer für die Behandlung von chronischer Veneninsuffizienz geeigneten Form.

## Claims

1. Diosmetin compounds of formula I: 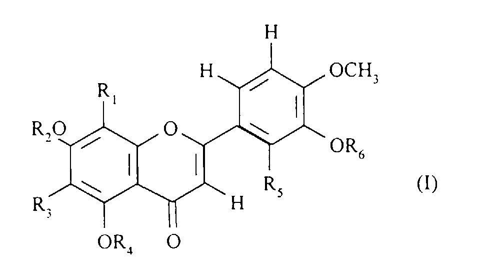 wherein :
- R₁ represents a hydrogen atom or a propyl, allyl or 1,2-dideuteroallyl radical ;
- R₂ represents a hydrogen atom or a propyl, allyl, propargyl, 2,3-dihydroxypropyl, (2,2-dimethyl-1,3-dioxol-4-yl)methyl or 3-acetoxy-2-hydroxypropyl radical;
- R₃ represents a hydrogen atom or a propyl, allyl or 1,2-dideuteroallyl radical ;
- R₄ represents a hydrogen atom, a methyl, propyl, allyl, propargyl, 2,3-dihydroxypropyl or (2,2-dimethyl-1,3-dioxol-4-yl)methyl radical or a radical of the formula -COR'₄ [wherein R'₄ represents an alkyl radical having from I to 5 carbon atoms in straight or branched chain or a phenyl radical];
- R₅ represents a hydrogen atom or a propyl, allyl or 1,2-dideuteroallyl radical, and
- R₆ represents a hydrogen atom, a methyl, propyl, allyl, propargyl, 2,3-dihydroxypropyl or (2,2-dimethyl-1,3-dioxol-4-yl)methyl radical, a radical of the formula -COR'₆ [wherein R'₆ represents an alkyl radical having from 1 to 5 carbon atoms in straight or branched chain or a phenyl radical] or a radical of formula A : 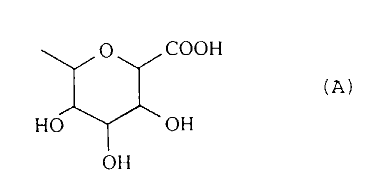 with the proviso that :
- at least onc of the radicals R₁, R₂, R₃, R₄, R₅ and R₆ has a meaning other than hydrogen, and that
- if R₁, R₂ and R₃ each simultaneously represent a hydrogen atom, then R₄ also represents a hydrogen atom;
- if R₁, R₂, R₃, R₄ and R₅ each simultaneously represent a hydrogen atom, then R₆ does not represent the radical of formula A;
and also their diastereoisomers and/or enantiomers.

2. A compound of claim 1, which is 7-allyloxy-5-hydroxy-2-(3-allyloxy-4-methoxyphenyl)-4H-1-benzopyran-4-one.

3. A compound of claim 1, which is 5,7-dihydroxy-2-{3-[(2,3-dihydroxypropoxy)-4-methoxyphenyl}-4H-1-benzopyran-4-one.

4. A compound of claim 1, which is (R,S)-5-hydroxy-2-(3-hydroxy-4-methoxyphenyl)-7-(2,3-dihydroxypropoxy)-4H-1-benzopyran-4-one.

5. A compound of claim 1, which is 5,7-di-(2,3-dihydroxypropoxy)-2-(3-hydroxy-4-methoxyphenyl)-4H-1-benzopyran-4-one.

6. A compound of claim 1, which is 5-hydroxy-2-(4-methoxy-3-pivaloyloxyphenyl)-7-(2,3-dihydroxypropoxy)-4H-1-benzopyran-4-one.

7. A compound of claim 1, which is 5-allyloxy-2-(3-allyloxy-4-methoxyphenyl)-7-(2,3-dihydroxypropoxy)-4H-1-benzopyran-4-one.

8. A compound of claim 1, which is 6-allyl-5-hydroxy-2-(2-allyl-3-hydroxy-4-methoxyphenyl)-7-(2,3-dihydroxypropoxy)-4H-1-benzopyran-4-one.

9. A compound of claim 1, which is 5-hydroxy-2-(3-hydroxy-4-methoxy-2-propylphenyl)-6-propyl-7-(2,3-dihydroxypropoxy)-4H-1-benzopyran-4-one.

10. A compound of claim 1, which is (R,S)-5-hydroxy-2-(3-hydroxy-4-methoxy-2-propylphenyl)-7-(7,3-dihydroxypropoxy)-4H-1-benzopyran-4-one, in the two forms α and β.

11. A compound of claim 1, which is (R)-5-hydroxy-2-(3-hydroxy-4-methoxy-2-propylphenyl)-7-(2,3-dihydroxypropoxy)-4H-1-benzopyran-4-one.

12. A compound of claim 1, which is (S)-5-hydroxy-2-(3-hydroxy-4-methoxy-2-propylphenyl)-7-(2,3-dihydroxypropoxy)-4H-1-benzopyran-4-one.

13. A compound of claim 1, which is (R,S)-5-hydroxy-2-(3-hydroxy-4-methoxy-2-propylphenyl)-7-(3-acetoxy-2-hydroxypropoxy)-4H-1-benzopyran-4-one.

14. A compound of claim 1, which is 5-hydroxy-2-[4-methoxy-2-propyl-3-(6-carboxy-3,4,5-trihydroxytetrahydropyran-2-yloxy)phenyl]-7-(2,3-dihydroxypropoxy)-4H-1-benzopyran-4-one.

15. A compound of claim 1, which is 5-hydroxy-2-[4-methoxy-3-(2,3-dihydroxypropoxy)phenyl]-7-(2,3-dihydroxypropoxy)-4H-1-benzopyran-4-one.

16. A compound of claim 1, which is 6,8-diallyl-5,7-dihydroxy-2-(2-allyl-3-hydroxy-4-methoxyphenyl)-4H-1-benzopyran-4-one.

17. A compound of claim 1, which is 5,7-dihydroxy-2-(3-hydroxy-4-methoxy-2-propylphenyl)-6,8-dipropyl-4H-1-benzopyran-4-one.

18. A process for the preparation of compounds of claim 1, characterised in that diosmetin of formula II: 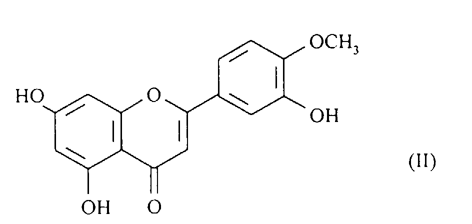 is treated :
A/ either with a compound of formula III : 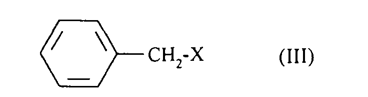 wherein X represents a halogen atom or a tosyloxy radical,
to obtain a compound of formula IV : 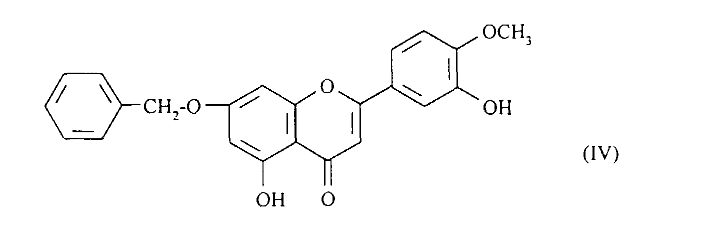 which is treated :
a) either with an allyl halide of formula H₂C=CH-CH₂-Hal wherein Hal represents a chlorine or bromine atom,
to yield a compound of formula V : 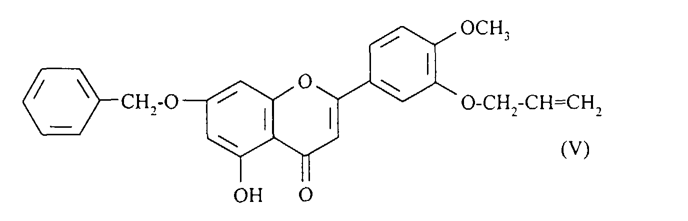 which, by transposition, yields the compound of formula VI : 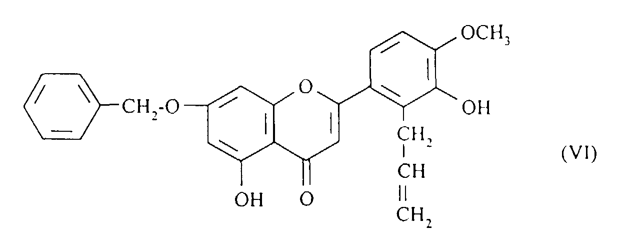 which, by hydrogenation, yields the compound of formula Ia : 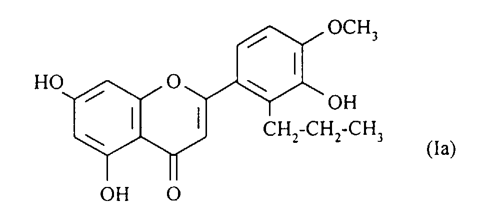 which, treated in turn with an allyl halide of formula H₂C=CH-CH₂-Hal wherein Hal represents a chlorine or bromine atom, yields the compound of formula (Ib) : 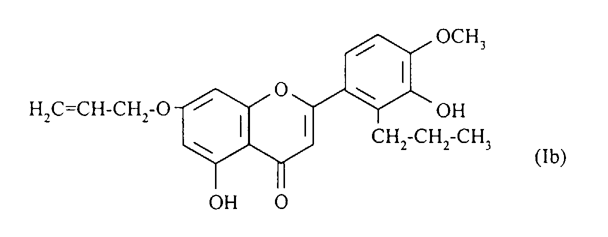 which, by hydrogenation, yields the compound of formula Ic : 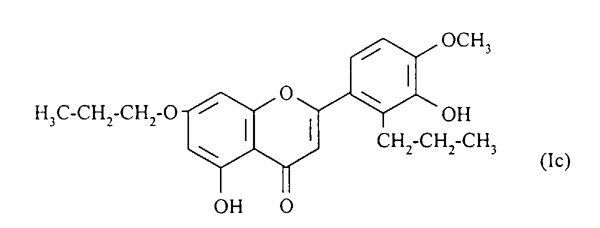
b) or with (2,2-dimethyl-1,3-dioxol-4-yl)methyl tosylate of formula : 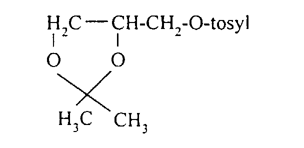 to yield the compound of formula VII : 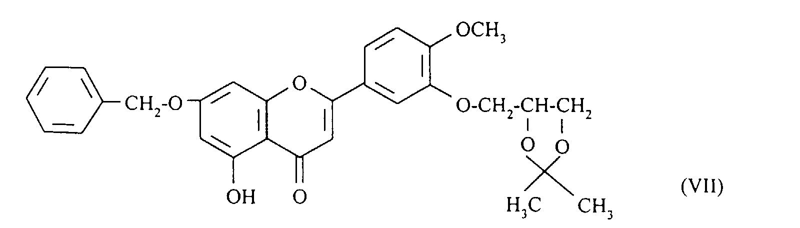 which, by hydrogenation, yields the compound of formula Id : 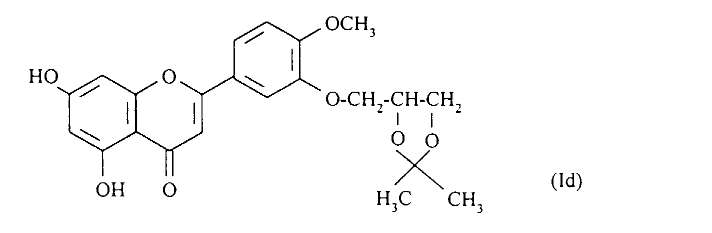 which, treated with acetic acid and H₂O yields the compound of formula Ie : 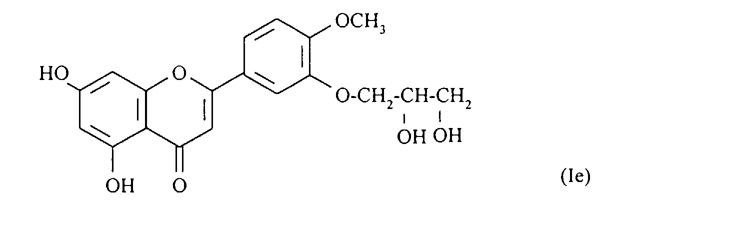 or
B/ with a compound of formula III' :
R'₂-X (III')
wherein :
- R'₂ represents an allyl, propargyl or (2,2-dimethyl-1,3-dioxol-4-yl)methyl radical, and
- X is as defined above, to obtain, depending on the quantity of compound III' in relation to that of compound II, one or other of the compounds of formulae If, Ig and Ih : 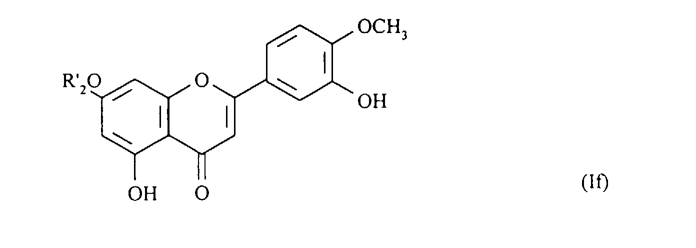 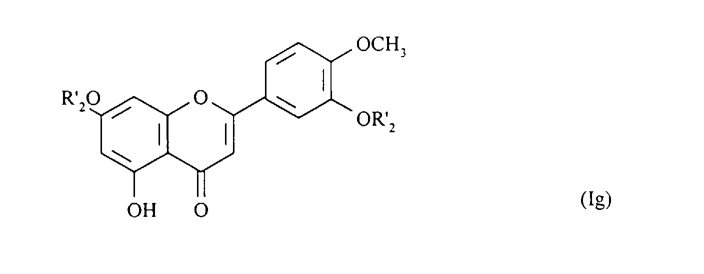 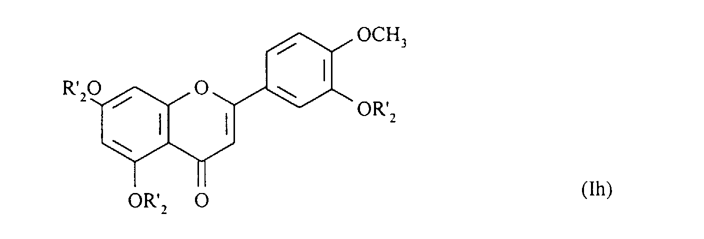
which compounds may be subjected to subsequent reactions selected from alkylation, transposition, esterification, reduction and hydrolysis to yield products of formula I other than those of formulae Ia to Ih the preparation of which is described above.

19. Pharmaceutical compositions comprising as active ingredient a compound according to any one of claims 1 to 17, mixed with or in association with one or more appropriate pharmaceutical excipients.

20. Pharmaceutical compositions according to claim 19 presented in a form suitable in the treatment of chronic venous insufficiency.
